# EUROPEAN PATENT APPLICATION

(11) **EP 3 925 615 A1**
(43) Date of publication of application: **22.12.2021**
(21) Application number: 21175876.8
(22) Date of filing: 10.10.2012
(51) Int. Cl.: A61K 38/20, A61K 39/395, A61K 45/06, A61P 35/00

(54) **COMBINATION MEDICAMENT COMPRISING IL-12 AND AN AGENT FOR BLOCKADE OF T-CELL INHIBITORY MOLECULES FOR TUMOUR THERAPY**

(30) Priority: 11.10.2011 EP 11184644; 10.11.2011 EP 11188625; 19.09.2012 EP 12185108
(62) Divisional of application: 18160057.8
(71) Applicant: Universität Zürich, 8006 Zürich (CH)
(72) Inventor: Becher, Prof. Burkhard, 8124 Maur (CH); vom Berg, Johannes, 8051 Zürich (CH)
(74) Representative: Schulz Junghans Patentanwälte PartGmbB

(57) **Abstract**

The invention relates to an IL-12 polypeptide, or a nucleic acid expression vector encoding an IL-12 polypeptide, for use in the treatment of a malignant neoplastic disease, provided by administration into a tumour, into the vicinity of a tumour, or to the lymph node associated with a tumour, wherein the IL-12 nucleic acid expression vector is administered in combination with a non-agonist ligand selected from the group consisting of an antibody, an antibody fragment, and an antibody-like molecule, wherein said non-agonist ligand is capable of binding to one of CTLA-4, PD-1, PD-L1 or PD-L2, provided as a dosage form for systemic delivery.

SEQUENCE LISTING

<110>  Universitaet Zuerich

<120>  Combination medicament comprising IL-12 and an agent for blockade of
T-cell inhibitory molecules for tumour therapy

<130>  uz117wo-ep2

<160>  9

<170>  PatentIn version 3.5

<210>  1
<211>  775
<212>  PRT
<213>  artificial

<220>
<223>  fusion between human IL-12 and IgG4 Fc

<400>  1

Met Cys His Gln Gln Leu Val Ile Ser Trp Phe Ser Leu Val Phe Leu
1               5                   10                  15

Ala Ser Pro Leu Val Ala Ile Trp Glu Leu Lys Lys Asp Val Tyr Val
            20                  25                  30

Val Glu Leu Asp Trp Tyr Pro Asp Ala Pro Gly Glu Met Val Val Leu
        35                  40                  45

Thr Cys Asp Thr Pro Glu Glu Asp Gly Ile Thr Trp Thr Leu Asp Gln
    50                  55                  60

Ser Ser Glu Val Leu Gly Ser Gly Lys Thr Leu Thr Ile Gln Val Lys
65                  70                  75                  80

Glu Phe Gly Asp Ala Gly Gln Tyr Thr Cys His Lys Gly Gly Glu Val
                85                  90                  95

Leu Ser His Ser Leu Leu Leu Leu His Lys Lys Glu Asp Gly Ile Trp
            100                 105                 110

Ser Thr Asp Ile Leu Lys Asp Gln Lys Glu Pro Lys Asn Lys Thr Phe
        115                 120                 125

Leu Arg Cys Glu Ala Lys Asn Tyr Ser Gly Arg Phe Thr Cys Trp Trp
    130                 135                 140

Leu Thr Thr Ile Ser Thr Asp Leu Thr Phe Ser Val Lys Ser Ser Arg
145                 150                 155                 160

Gly Ser Ser Asp Pro Gln Gly Val Thr Cys Gly Ala Ala Thr Leu Ser
                165                 170                 175

Ala Glu Arg Val Arg Gly Asp Asn Lys Glu Tyr Glu Tyr Ser Val Glu
            180                 185                 190

Cys Gln Glu Asp Ser Ala Cys Pro Ala Ala Glu Glu Ser Leu Pro Ile
        195                 200                 205

Glu Val Met Val Asp Ala Val His Lys Leu Lys Tyr Glu Asn Tyr Thr
    210                 215                 220

Ser Ser Phe Phe Ile Arg Asp Ile Ile Lys Pro Asp Pro Pro Lys Asn
225                 230                 235                 240

Leu Gln Leu Lys Pro Leu Lys Asn Ser Arg Gln Val Glu Val Ser Trp
                245                 250                 255

Glu Tyr Pro Asp Thr Trp Ser Thr Pro His Ser Tyr Phe Ser Leu Thr
            260                 265                 270

Phe Cys Val Gln Val Gln Gly Lys Ser Lys Arg Glu Lys Lys Asp Arg
        275                 280                 285

Val Phe Thr Asp Lys Thr Ser Ala Thr Val Ile Cys Arg Lys Asn Ala
    290                 295                 300

Ser Ile Ser Val Arg Ala Gln Asp Arg Tyr Tyr Ser Ser Ser Trp Ser
305                 310                 315                 320

Glu Trp Ala Ser Val Pro Cys Ser Gly Gly Gly Gly Ser Gly Gly Gly
                325                 330                 335

Gly Ser Gly Gly Gly Gly Ser Arg Asn Leu Pro Val Ala Thr Pro Asp
            340                 345                 350

Pro Gly Met Phe Pro Cys Leu His His Ser Gln Asn Leu Leu Arg Ala
        355                 360                 365

Val Ser Asn Met Leu Gln Lys Ala Arg Gln Thr Leu Glu Phe Tyr Pro
    370                 375                 380

Cys Thr Ser Glu Glu Ile Asp His Glu Asp Ile Thr Lys Asp Lys Thr
385                 390                 395                 400

Ser Thr Val Glu Ala Cys Leu Pro Leu Glu Leu Thr Lys Asn Glu Ser
                405                 410                 415

Cys Leu Asn Ser Arg Glu Thr Ser Phe Ile Thr Asn Gly Ser Cys Leu
                420                 425                 430

Ala Ser Arg Lys Thr Ser Phe Met Met Ala Leu Cys Leu Ser Ser Ile
            435                 440                 445

Tyr Glu Asp Leu Lys Met Tyr Gln Val Glu Phe Lys Thr Met Asn Ala
    450                 455                 460

Lys Leu Leu Met Asp Pro Lys Arg Gln Ile Phe Leu Asp Gln Asn Met
465                 470                 475                 480

Leu Ala Val Ile Asp Glu Leu Met Gln Ala Leu Asn Phe Asn Ser Glu
                485                 490                 495

Thr Val Pro Gln Lys Ser Ser Leu Glu Glu Pro Asp Phe Tyr Lys Thr
            500                 505                 510

Lys Ile Lys Leu Cys Ile Leu Leu His Ala Phe Arg Ile Arg Ala Val
        515                 520                 525

Thr Ile Asp Arg Val Met Ser Tyr Leu Asn Ala Ser Lys Val Asp Lys
    530                 535                 540

Arg Val Glu Ser Lys Tyr Gly Pro Pro Cys Pro Ser Cys Pro Ala Pro
545                 550                 555                 560

Glu Phe Leu Gly Gly Pro Ser Val Phe Leu Phe Pro Pro Lys Pro Lys
                565                 570                 575

Asp Thr Leu Met Ile Ser Arg Thr Pro Glu Val Thr Cys Val Val Val
            580                 585                 590

Asp Val Ser Gln Glu Asp Pro Glu Val Gln Phe Asn Trp Tyr Val Asp
        595                 600                 605

Gly Val Glu Val His Asn Ala Lys Thr Lys Pro Arg Glu Glu Gln Phe
    610                 615                 620

Asn Ser Thr Tyr Arg Val Val Ser Val Leu Thr Val Leu His Gln Asp
625                 630                 635                 640

Trp Leu Asn Gly Lys Glu Tyr Lys Cys Lys Val Ser Asn Lys Gly Leu
                645                 650                 655

Pro Ser Ser Ile Glu Lys Thr Ile Ser Lys Ala Lys Gly Gln Pro Arg
            660                 665                 670

Glu Pro Gln Val Tyr Thr Leu Pro Pro Ser Pro Glu Glu Met Thr Lys
        675                 680                 685

Asn Gln Val Ser Leu Thr Cys Leu Val Lys Gly Phe Tyr Pro Ser Asp
    690                 695                 700

Ile Ala Val Glu Trp Glu Ser Asn Gly Gln Pro Glu Asn Asn Tyr Lys
705                 710                 715                 720

Thr Thr Pro Pro Val Leu Asp Ser Asp Gly Ser Phe Phe Leu Tyr Ser
                725                 730                 735

Arg Leu Thr Val Asp Lys Ser Arg Trp Gln Glu Gly Asn Val Phe Ser
            740                 745                 750

Cys Ser Val Met His Glu Ala Leu His Asn His Tyr Thr Gln Lys Ser
        755                 760                 765

Leu Ser Leu Ser Leu Gly Lys
    770                 775

<210>  2
<211>  781
<212>  PRT
<213>  Artificial Sequence

<220>
<223>  murine IL-12 IgG3 Fc fusion construct

<400>  2

Met Cys Pro Gln Lys Leu Thr Ile Ser Trp Phe Ala Ile Val Leu Leu
1               5                   10                  15

Val Ser Pro Leu Met Ala Met Trp Glu Leu Glu Lys Asp Val Tyr Val
            20                  25                  30

Val Glu Val Asp Trp Thr Pro Asp Ala Pro Gly Glu Thr Val Asn Leu
        35                  40                  45

Thr Cys Asp Thr Pro Glu Glu Asp Asp Ile Thr Trp Thr Ser Asp Gln
    50                  55                  60

Arg His Gly Val Ile Gly Ser Gly Lys Thr Leu Thr Ile Thr Val Lys
65                  70                  75                  80

Glu Phe Leu Asp Ala Gly Gln Tyr Thr Cys His Lys Gly Gly Glu Thr
                85                  90                  95

Leu Ser His Ser His Leu Leu Leu His Lys Lys Glu Asn Gly Ile Trp
        100                 105                 110

Ser Thr Glu Ile Leu Lys Asn Phe Lys Asn Lys Thr Phe Leu Lys Cys
    115                 120                 125

Glu Ala Pro Asn Tyr Ser Gly Arg Phe Thr Cys Ser Trp Leu Val Gln
    130                 135                 140

Arg Asn Met Asp Leu Lys Phe Asn Ile Lys Ser Ser Ser Ser Ser Pro
145                 150                 155                 160

Asp Ser Arg Ala Val Thr Cys Gly Met Ala Ser Leu Ser Ala Glu Lys
                165                 170                 175

Val Thr Leu Asp Gln Arg Asp Tyr Glu Lys Tyr Ser Val Ser Cys Gln
            180                 185                 190

Glu Asp Val Thr Cys Pro Thr Ala Glu Glu Thr Leu Pro Ile Glu Leu
        195                 200                 205

Ala Leu Glu Ala Arg Gln Gln Asn Lys Tyr Glu Asn Tyr Ser Thr Ser
    210                 215                 220

Phe Phe Ile Arg Asp Ile Ile Lys Pro Asp Pro Pro Lys Asn Leu Gln
225                 230                 235                 240

Met Lys Pro Leu Lys Asn Ser Gln Val Glu Val Ser Trp Glu Tyr Pro
                245                 250                 255

Asp Ser Trp Ser Thr Pro His Ser Tyr Phe Ser Leu Lys Phe Phe Val
            260                 265                 270

Arg Ile Gln Arg Lys Lys Glu Lys Met Lys Glu Thr Glu Glu Gly Cys
        275                 280                 285

Asn Gln Lys Gly Ala Phe Leu Val Glu Lys Thr Ser Thr Glu Val Gln
    290                 295                 300

Cys Lys Gly Gly Asn Val Cys Val Gln Ala Gln Asp Arg Tyr Tyr Asn
305                 310                 315                 320

Ser Ser Cys Ser Lys Trp Ala Cys Val Pro Cys Arg Val Arg Ser Gly
                325                 330                 335

Gly Gly Gly Ser Gly Gly Gly Gly Ser Gly Gly Gly Gly Ser Arg Val
            340                 345                 350

Ile Pro Val Ser Gly Pro Ala Arg Cys Leu Ser Gln Ser Arg Asn Leu
        355                 360                 365

Leu Lys Thr Thr Asp Asp Met Val Lys Thr Ala Arg Glu Lys Leu Lys
    370                 375                 380

His Tyr Ser Cys Thr Ala Glu Asp Ile Asp His Glu Asp Ile Thr Arg
385                 390                 395                 400

Asp Gln Thr Ser Thr Leu Lys Thr Cys Leu Pro Leu Glu Leu His Lys
                405                 410                 415

Asn Glu Ser Cys Leu Ala Thr Arg Glu Thr Ser Ser Thr Thr Arg Gly
            420                 425                 430

Ser Cys Leu Pro Pro Gln Lys Thr Ser Leu Met Met Thr Leu Cys Leu
        435                 440                 445

Gly Ser Ile Tyr Glu Asp Leu Lys Met Tyr Gln Thr Glu Phe Gln Ala
    450                 455                 460

Ile Asn Ala Ala Leu Gln Asn His Asn His Gln Gln Ile Ile Leu Asp
465                 470                 475                 480

Lys Gly Met Leu Val Ala Ile Asp Glu Leu Met Gln Ser Leu Asn His
                485                 490                 495

Asn Gly Glu Thr Leu Arg Gln Lys Pro Pro Val Gly Glu Ala Asp Pro
            500                 505                 510

Tyr Arg Val Lys Met Lys Leu Cys Ile Leu Leu His Ala Phe Ser Thr
        515                 520                 525

Arg Val Val Thr Ile Asn Arg Val Met Gly Tyr Leu Ser Ser Ala Leu
    530                 535                 540

Ile Lys Arg Ile Glu Pro Arg Ile Pro Lys Pro Ser Thr Pro Pro Gly
545                 550                 555                 560

Ser Ser Cys Pro Pro Gly Asn Ile Leu Gly Gly Pro Ser Val Phe Ile
                565                 570                 575

Phe Pro Pro Lys Pro Lys Asp Ala Leu Met Ile Ser Leu Thr Pro Lys
            580                 585                 590

Val Thr Cys Val Val Val Asp Val Ser Glu Asp Asp Pro Asp Val His

595                 600                 605

Val Ser Trp Phe Val Asp Asn Lys Glu Val His Thr Ala Trp Thr Gln
    610                 615                 620

Pro Arg Glu Ala Gln Tyr Asn Ser Thr Phe Arg Val Val Ser Ala Leu
625                 630                 635                 640

Pro Ile Gln His Gln Asp Trp Met Arg Gly Lys Glu Phe Lys Cys Lys
                645                 650                 655

Val Asn Asn Lys Ala Leu Pro Ala Pro Ile Glu Arg Thr Ile Ser Lys
            660                 665                 670

Pro Lys Gly Arg Ala Gln Thr Pro Gln Val Tyr Thr Ile Pro Pro Pro
        675                 680                 685

Arg Glu Gln Met Ser Lys Lys Lys Val Ser Leu Thr Cys Leu Val Thr
    690                 695                 700

Asn Phe Phe Ser Glu Ala Ile Ser Val Glu Trp Glu Arg Asn Gly Glu
705                 710                 715                 720

Leu Glu Gln Asp Tyr Lys Asn Thr Pro Pro Ile Leu Asp Ser Asp Gly
                725                 730                 735

Thr Tyr Phe Leu Tyr Ser Lys Leu Thr Val Asp Thr Asp Ser Trp Leu
            740                 745                 750

Gln Gly Glu Ile Phe Thr Cys Ser Val Val His Glu Ala Leu His Asn
        755                 760                 765

His His Thr Gln Lys Asn Leu Ser Arg Ser Pro Gly Lys
    770                 775                 780

<210>  3
<211>  17
<212>  DNA
<213>  artificial

<220>
<223>  PCR primer

<400>  3
acacacagcc tggacgc                                                    17

<210>  4
<211>  20
<212>  DNA
<213>  artificial

<220>
<223>  PCR primer

<400>  4
catttgaact ccttgcccct                                                   20


<210>  5
<211>  219
<212>  PRT
<213>  homo sapiens

<400>  5

Met Cys Pro Ala Arg Ser Leu Leu Leu Val Ala Thr Leu Val Leu Leu
1               5                   10                  15


Asp His Leu Ser Leu Ala Arg Asn Leu Pro Val Ala Thr Pro Asp Pro
            20                  25                  30


Gly Met Phe Pro Cys Leu His His Ser Gln Asn Leu Leu Arg Ala Val
        35                  40                  45


Ser Asn Met Leu Gln Lys Ala Arg Gln Thr Leu Glu Phe Tyr Pro Cys
    50                  55                  60


Thr Ser Glu Glu Ile Asp His Glu Asp Ile Thr Lys Asp Lys Thr Ser
65                  70                  75                  80


Thr Val Glu Ala Cys Leu Pro Leu Glu Leu Thr Lys Asn Glu Ser Cys
                85                  90                  95


Leu Asn Ser Arg Glu Thr Ser Phe Ile Thr Asn Gly Ser Cys Leu Ala
            100                 105                 110


Ser Arg Lys Thr Ser Phe Met Met Ala Leu Cys Leu Ser Ser Ile Tyr
        115                 120                 125


Glu Asp Leu Lys Met Tyr Gln Val Glu Phe Lys Thr Met Asn Ala Lys
    130                 135                 140


Leu Leu Met Asp Pro Lys Arg Gln Ile Phe Leu Asp Gln Asn Met Leu
145                 150                 155                 160


Ala Val Ile Asp Glu Leu Met Gln Ala Leu Asn Phe Asn Ser Glu Thr
                165                 170                 175


Val Pro Gln Lys Ser Ser Leu Glu Glu Pro Asp Phe Tyr Lys Thr Lys
            180                 185                 190

Ile Lys Leu Cys Ile Leu Leu His Ala Phe Arg Ile Arg Ala Val Thr
        195                 200                 205

Ile Asp Arg Val Met Ser Tyr Leu Asn Ala Ser
    210                 215

<210>  6
<211>  328
<212>  PRT
<213>  homo sapiens

<400>  6

Met Cys His Gln Gln Leu Val Ile Ser Trp Phe Ser Leu Val Phe Leu
1               5                   10                  15

Ala Ser Pro Leu Val Ala Ile Trp Glu Leu Lys Lys Asp Val Tyr Val
            20                  25                  30

Val Glu Leu Asp Trp Tyr Pro Asp Ala Pro Gly Glu Met Val Val Leu
        35                  40                  45

Thr Cys Asp Thr Pro Glu Glu Asp Gly Ile Thr Trp Thr Leu Asp Gln
    50                  55                  60

Ser Ser Glu Val Leu Gly Ser Gly Lys Thr Leu Thr Ile Gln Val Lys
65                  70                  75                  80

Glu Phe Gly Asp Ala Gly Gln Tyr Thr Cys His Lys Gly Gly Glu Val
                85                  90                  95

Leu Ser His Ser Leu Leu Leu Leu His Lys Lys Glu Asp Gly Ile Trp
            100                 105                 110

Ser Thr Asp Ile Leu Lys Asp Gln Lys Glu Pro Lys Asn Lys Thr Phe
        115                 120                 125

Leu Arg Cys Glu Ala Lys Asn Tyr Ser Gly Arg Phe Thr Cys Trp Trp
    130                 135                 140

Leu Thr Thr Ile Ser Thr Asp Leu Thr Phe Ser Val Lys Ser Ser Arg
145                 150                 155                 160

Gly Ser Ser Asp Pro Gln Gly Val Thr Cys Gly Ala Ala Thr Leu Ser
                165                 170                 175

Ala Glu Arg Val Arg Gly Asp Asn Lys Glu Tyr Glu Tyr Ser Val Glu
            180                 185                 190

Cys Gln Glu Asp Ser Ala Cys Pro Ala Ala Glu Glu Ser Leu Pro Ile
        195                 200                 205

Glu Val Met Val Asp Ala Val His Lys Leu Lys Tyr Glu Asn Tyr Thr
    210                 215                 220

Ser Ser Phe Phe Ile Arg Asp Ile Ile Lys Pro Asp Pro Pro Lys Asn
225                 230                 235                 240

Leu Gln Leu Lys Pro Leu Lys Asn Ser Arg Gln Val Glu Val Ser Trp
                245                 250                 255

Glu Tyr Pro Asp Thr Trp Ser Thr Pro His Ser Tyr Phe Ser Leu Thr
            260                 265                 270

Phe Cys Val Gln Val Gln Gly Lys Ser Lys Arg Glu Lys Lys Asp Arg
        275                 280                 285

Val Phe Thr Asp Lys Thr Ser Ala Thr Val Ile Cys Arg Lys Asn Ala
    290                 295                 300

Ser Ile Ser Val Arg Ala Gln Asp Arg Tyr Tyr Ser Ser Ser Trp Ser
305                 310                 315                 320

Glu Trp Ala Ser Val Pro Cys Ser
                325

<210>  7
<211>  2328
<212>  DNA
<213>  artificial

<220>
<223>  expression construct, coding sequence for IL-12 IgG4 Fc fusion
       protein

<400>  7
atgtgtcacc agcagttggt catctcttgg ttttccctgg tttttctggc atctcccctc      60

gtggccatat gggaactgaa gaaagatgtt tatgtcgtag aattggattg gtatccggat     120

gcccctggag aaatggtggt cctcacctgt gacacccctg aagaagatgg tatcacctgg     180

accttggacc agagcagtga ggtcttaggc tctggcaaaa ccctgaccat ccaagtcaaa     240

gagtttggag atgctggcca gtacacctgt cacaaaggag gcgaggttct aagccattcg     300

ctcctgctgc ttcacaaaaa ggaagatgga atttggtcca ctgatatttt aaaggaccag     360

aaagaaccca aaaataagac ctttctaaga tgcgaggcca agaattattc tggacgtttc     420

acctgctggt ggctgacgac aatcagtact gatttgacat tcagtgtcaa aagcagcaga     480

ggctcttctg acccccaagg ggtgacgtgc ggagctgcta cactctctgc agagagagtc     540

agaggggaca acaaggagta tgagtactca gtggagtgcc aggaggacag tgcctgccca 600

gctgctgagg agagtctgcc cattgaggtc atggtggatg ccgttcacaa gctcaagtat 660

gaaaactaca ccagcagctt cttcatcagg gacatcatca aacctgaccc acccaagaac 720

ttgcagctga agccattaaa gaattctcgg caggtggagg tcagctggga gtaccctgac 780

acctggagta ctccacattc ctacttctcc ctgacattct gcgttcaggt ccagggcaag 840

agcaagagag aaaagaaaga tagagtcttc acggacaaga cctcagccac ggtcatctgc 900

cgcaaaaatg ccagcattag cgtgcgggcc caggaccgct actatagctc atcttggagc 960

gaatgggcat ctgtgccctg cagtggaggc ggtggctcgg gcggtggtgg gtcgggtggc 1020

ggcggatcca gaaacctccc cgtggccact ccagacccag gaatgttccc atgccttcac 1080

cactcccaaa acctgctgag ggccgtcagc aacatgctcc agaaggccag acaaactcta 1140

gaattttacc cttgcacttc tgaagagatt gatcatgaag atatcacaaa agataaaacc 1200

agcacagtgg aggcctgttt accattggaa ttaaccaaga atgagagttg cctaaattcc 1260

agagagacct ctttcataac taatgggagt tgcctggcct ccagaaagac ctcttttatg 1320

atggccctgt gccttagtag tatttatgaa gacttgaaga tgtaccaggt ggagttcaag 1380

accatgaatg caaagcttct gatggatcct aagaggcaga tctttctaga tcaaaacatg 1440

ctggcagtta ttgatgagct gatgcaggcc ctgaatttca acagtgagac tgtgccacaa 1500

aaatcctccc ttgaagaacc ggatttttat aaaactaaaa tcaagctctg catacttctt 1560

catgctttca gaattcgggc agtgactatt gatagagtga tgagctatct gaatgcttcc 1620

aaggtggaca agagagttga gtccaaatat ggtcccccat gcccatcatg cccagcacct 1680

gagttcctgg ggggaccatc agtcttcctg ttccccccaa aacccaagga cactctcatg 1740

atctcccgga cccctgaggt cacgtgcgtg gtggtggacg tgagccagga agaccccgag 1800

gtccagttca actggtacgt ggatggcgtg gaggtgcata atgccaagac aaagccgcgg 1860

gaggagcagt tcaacagcac gtaccgtgtg gtcagcgtcc tcaccgtcct gcaccaggac 1920

tggctgaacg gcaaggagta caagtgcaag gtctccaaca aaggcctccc gtcctccatc 1980

gagaaaacca tctccaaagc caaagggcag ccccgagagc cacaggtgta caccctgccc 2040

ccatccccgg aggagatgac caagaaccag gtcagcctga cctgcctggt caaaggcttc 2100

taccccagcg acatcgccgt ggagtgggag agcaatgggc agccggagaa caactacaag 2160

accacgcctc ccgtgctgga ctccgacggc tccttcttcc tctacagcag gctaaccgtg 2220

gacaagagca ggtggcagga ggggaatgtc ttctcatgct ccgtgatgca tgaggctctg 2280

cacaaccact acacacagaa gagcctctcc ctgtctctgg gtaaatga 2328

<210> 8

<211>  10994
<212>  DNA
<213>  artificial

<220>
<223>  plasmid vector encoding Fc tag (murine)

<400>  8
ctcgcagcaa agcaagatgt gtcctcagaa gctaaccatc tcctggtttg ccatcgtttt      60

gctggtgtct ccactcatgg ccatgtggga gctggagaag cttatcaaga gaatcgagcc     120

tagaataccc aagcccagta cccccccagg ttcttcatgc ccacctggta acatcttggg     180

tggaccatcc gtcttcatct tccccccaaa gcccaaggat gcactcatga tctccctaac     240

ccccaaggtt acgtgtgtgg tggtggatgt gagcgaggat gacccagatg tccatgtcag     300

ctggtttgtg gacaacaaag aagtacacac agcctggacg cagccccgtg aagctcagta     360

caacagtacc ttccgagtgg tcagtgccct ccccatccag caccaggact ggatgagggg     420

caaggagttc aaatgcaagg tcaacaacaa agccctccca gcccccatcg agagaaccat     480

ctcaaaaccc aaaggaagag cccagacacc tcaagtatac accatacccc cacctcgtga     540

acaaatgtcc aagaagaagg ttagtctgac ctgcctggtc accaacttct tctctgaagc     600

catcagtgtg gagtgggaaa ggaacggaga actggagcag gattacaaga acactccacc     660

catcctggac tcggatggga cctacttcct ctacagcaag ctcactgtgg atacagacag     720

ttggttgcaa ggagaaattt ttacctgctc cgtggtgcat gaggctctcc ataaccacca     780

cacacagaag aacctgtctc gctcccctgg taaatgagaa cagcatctag cggccgctcg     840

aggccggcaa ggccggatcc agacatgata agatacattg atgagtttgg acaaaccaca     900

actagaatgc agtgaaaaaa atgctttatt tgtgaaattt gtgatgctat tgctttattt     960

gtaaccatta taagctgcaa taaacaagtt aacaacaaca attgcattca ttttatgttt    1020

caggttcagg gggaggtgtg ggaggttttt taaagcaagt aaaacctcta caaatgtggt    1080

atggctgatt atgatccggc tgcctcgcgc gtttcggtga tgacggtgaa aacctctgac    1140

acatgcagct cccggagacg gtcacagctt gtctgtaagc ggatgccggg agcagacaag    1200

cccgtcaggc gtcagcgggt gttggcgggt gtcggggcgc agccatgagg tcgactctag    1260

aggatcgatg ccccgccccg gacgaactaa acctgactac gacatctctg ccccttcttc    1320

gcggggcagt gcatgtaatc ccttcagttg gttggtacaa cttgccaact gggccctgtt    1380

ccacatgtga cacggggggg gaccaaacac aaaggggttc tctgactgta gttgacatcc    1440

ttataaatgg atgtgcacat ttgccaacac tgagtggctt tcatcctgga gcagactttg    1500

cagtctgtgg actgcaacac aacattgcct ttatgtgtaa ctcttggctg aagctcttac    1560

accaatgctg ggggacatgt acctcccagg ggcccaggaa gactacggga ggctacacca    1620

acgtcaatca gaggggcctg tgtagctacc gataagcgga ccctcaagag ggcattagca    1680

atagtgttta taaggccccc ttgttaaccc taaacgggta gcatatgctt cccgggtagt 1740

agtatatact atccagacta accctaattc aatagcatat gttacccaac gggaagcata 1800

tgctatcgaa ttagggttag taaaagggtc ctaaggaaca gcgatatctc ccaccccatg 1860

agctgtcacg gttttattta catggggtca ggattccacg agggtagtga accattttag 1920

tcacaagggc agtggctgaa gatcaaggag cgggcagtga actctcctga atcttcgcct 1980

gcttcttcat tctccttcgt ttagctaata gaataactgc tgagttgtga acagtaaggt 2040

gtatgtgagg tgctcgaaaa caaggtttca ggtgacgccc ccagaataaa atttggacgg 2100

ggggttcagt ggtggcattg tgctatgaca ccaatataac cctcacaaac cccttgggca 2160

ataaatacta gtgtaggaat gaaacattct gaatatcttt aacaatagaa atccatgggg 2220

tggggacaag ccgtaaagac tggatgtcca tctcacacga atttatggct atgggcaaca 2280

cataatccta gtgcaatatg atactggggt tattaagatg tgtcccaggc agggaccaag 2340

acaggtgaac catgttgtta cactctattt gtaacaaggg gaaagagagt ggacgccgac 2400

agcagcggac tccactggtt gtctctaaca cccccgaaaa ttaaacgggg ctccacgcca 2460

atggggccca taaacaaaga caagtggcca ctcttttttt tgaaattgtg gagtgggggc 2520

acgcgtcagc ccccacacgc cgccctgcgg ttttggactg taaaataagg gtgtaataac 2580

ttggctgatt gtaaccccgc taaccactgc ggtcaaacca cttgcccaca aaaccactaa 2640

tggcaccccg gggaatacct gcataagtag gtgggcgggc caagataggg gcgcgattgc 2700

tgcgatctgg aggacaaatt acacacactt gcgcctgagc gccaagcaca gggttgttgg 2760

tcctcatatt cacgaggtcg ctgagagcac ggtgggctaa tgttgccatg ggtagcatat 2820

actacccaaa tatctggata gcatatgcta tcctaatcta tatctgggta gcataggcta 2880

tcctaatcta tatctgggta gcatatgcta tcctaatcta tatctgggta gtatatgcta 2940

tcctaattta tatctgggta gcataggcta tcctaatcta tatctgggta gcatatgcta 3000

tcctaatcta tatctgggta gtatatgcta tcctaatctg tatccgggta gcatatgcta 3060

tcctaataga gattagggta gtatatgcta tcctaattta tatctgggta gcatatacta 3120

cccaaatatc tggatagcat atgctatcct aatctatatc tgggtagcat atgctatcct 3180

aatctatatc tgggtagcat aggctatcct aatctatatc tgggtagcat atgctatcct 3240

aatctatatc tgggtagtat atgctatcct aatttatatc tgggtagcat aggctatcct 3300

aatctatatc tgggtagcat atgctatcct aatctatatc tgggtagtat atgctatcct 3360

aatctgtatc cgggtagcat atgctatcct catgcatata cagtcagcat atgataccca 3420

gtagtagagt gggagtgcta tcctttgcat atgccgccac ctcccaaggg ggcgtgaatt 3480

ttcgctgctt gtccttttcc tgctggttgc tcccattctt aggtgaattt aaggaggcca 3540

ggctaaagcc gtcgcatgtc tgattgctca ccaggtaaat gtcgctaatg ttttccaacg 3600
cgagaaggtg ttgagcgcgg agctgagtga cgtgacaaca tgggtatgcc caattgcccc 3660
atgttgggag gacgaaaatg gtgacaagac agatggccag aaatacacca acagcacgca 3720
tgatgtctac tggggattta ttctttagtg cgggggaata cacggctttt aatacgattg 3780
agggcgtctc ctaacaagtt acatcactcc tgcccttcct caccctcatc tccatcacct 3840
ccttcatctc cgtcatctcc gtcatcaccc tccgcggcag ccccttccac cataggtgga 3900
aaccagggag gcaaatctac tccatcgtca aagctgcaca cagtcaccct gatattgcag 3960
gtaggagcgg gctttgtcat aacaaggtcc ttaatcgcat ccttcaaaac ctcagcaaat 4020
atatgagttt gtaaaaagac catgaaataa cagacaatgg actcccttag cgggccaggt 4080
tgtgggccgg gtccaggggc cattccaaag gggagacgac tcaatggtgt aagacgacat 4140
tgtggaatag caagggcagt tcctcgcctt aggttgtaaa gggaggtctt actacctcca 4200
tatacgaaca caccggcgac ccaagttcct tcgtcggtag tcctttctac gtgactccta 4260
gccaggagag ctcttaaacc ttctgcaatg ttctcaaatt tcgggttgga acctccttga 4320
ccacgatgct ttccaaacca ccctcctttt ttgcgcctgc ctccatcacc ctgaccccgg 4380
ggtccagtgc ttgggccttc tcctgggtca tctgcggggc cctgctctat cgctcccggg 4440
ggcacgtcag gctcaccatc tgggccacct tcttggtggt attcaaaata atcggcttcc 4500
cctacagggt ggaaaaatgg ccttctacct ggagggggcc tgcgcggtgg agacccggat 4560
gatgatgact gactactggg actcctgggc ctcttttctc cacgtccacg acctctcccc 4620
ctggctcttt cacgacttcc ccccctggct ctttcacgtc ctctaccccg gcggcctcca 4680
ctacctcctc gaccccggcc tccactacct cctcgacccc ggcctccact gcctcctcga 4740
ccccggcctc cacctcctgc tcctgcccct cctgctcctg cccctcctcc tgctcctgcc 4800
cctcctgccc ctcctgctcc tgcccctcct gcccctcctg ctcctgcccc tcctgcccct 4860
cctgctcctg cccctcctgc ccctcctcct gctcctgccc ctcctgcccc tcctcctgct 4920
cctgcccctc ctgcccctcc tgctcctgcc cctcctgccc ctcctgctcc tgcccctcct 4980
gcccctcctg ctcctgcccc tcctgctcct gcccctcctg ctcctgcccc tcctgctcct 5040
gcccctcctg cccctcctgc ccctcctcct gctcctgccc ctcctgctcc tgcccctcct 5100
gcccctcctg cccctcctgc tcctgcccct cctcctgctc ctgcccctcc tgcccctcct 5160
gcccctcctc ctgctcctgc ccctcctgcc cctcctcctg ctcctgcccc tcctcctgct 5220
cctgcccctc ctgcccctcc tgcccctcct cctgctcctg cccctcctgc ccctcctcct 5280
gctcctgccc ctcctcctgc tcctgcccct cctgcccctc ctgcccctcc tcctgctcct 5340
gcccctcctc ctgctcctgc ccctcctgcc cctcctgccc ctcctgcccc tcctcctgct 5400
cctgcccctc ctcctgctcc tgcccctcct gctcctgccc ctcccgctcc tgctcctgct 5460

cctgttccac cgtgggtccc tttgcagcca atgcaacttg gacgtttttg gggtctccgg 5520

acaccatctc tatgtcttgg ccctgatcct gagccgcccg gggctcctgg tcttccgcct 5580

cctcgtcctc gtcctcttcc ccgtcctcgt ccatggttat caccccctct tctttgaggt 5640

ccactgccgc cggagccttc tggtccagat gtgtctccct tctctcctag gccatttcca 5700

ggtcctgtac ctggcccctc gtcagacatg attcacacta aaagagatca atagacatct 5760

ttattagacg acgctcagtg aatacaggga gtgcagactc ctgccccctc caacagcccc 5820

cccaccctca tccccttcat ggtcgctgtc agacagatcc aggtctgaaa attccccatc 5880

ctccgaacca tcctcgtcct catcaccaat tactcgcagc ccggaaaact cccgctgaac 5940

atcctcaaga tttgcgtcct gagcctcaag ccaggcctca aattcctcgt cccccttttt 6000

gctggacggt agggatgggg attctcggga cccctcctct tcctcttcaa ggtcaccaga 6060

cagagatgct actggggcaa cggaagaaaa gctgggtgcg gcctgtgagg atcagcttat 6120

cgatgataag ctgtcaaaca tgagaattct tgaagacgaa agggcctcgt gatacgccta 6180

tttttatagg ttaatgtcat gataataatg gtttcttaga cgtcaggtgg cacttttcgg 6240

ggaaatgtgc gcggaacccc tatttgttta tttttctaaa tacattcaaa tatgtatccg 6300

ctcatgagac aataaccctg ataaatgctt caataatatt gaaaaaggaa gagtatgagt 6360

attcaacatt tccgtgtcgc ccttattccc ttttttgcgg cattttgcct tcctgttttt 6420

gctcacccag aaacgctggt gaaagtaaaa gatgctgaag atcagttggg tgcacgagtg 6480

ggttacatcg aactggatct caacagcggt aagatccttg agagttttcg ccccgaagaa 6540

cgttttccaa tgatgagcac ttttaaagtt ctgctatgtg gcgcggtatt atcccgtgtt 6600

gacgccgggc aagagcaact cggtcgccgc atacactatt ctcagaatga cttggttgag 6660

tactcaccag tcacagaaaa gcatcttacg gatggcatga cagtaagaga attatgcagt 6720

gctgccataa ccatgagtga taacactgcg gccaacttac ttctgacaac gatcggagga 6780

ccgaaggagc taaccgcttt tttgcacaac atgggggatc atgtaactcg ccttgatcgt 6840

tgggaaccgg agctgaatga agccatacca aacgacgagc gtgacaccac gatgcctgca 6900

gcaatggcaa caacgttgcg caaactatta actggcgaac tacttactct agcttcccgg 6960

caacaattaa tagactggat ggaggcggat aaagttgcag gaccacttct gcgctcggcc 7020

cttccggctg gctggtttat tgctgataaa tctggagccg gtgagcgtgg gtctcgcggt 7080

atcattgcag cactggggcc agatggtaag ccctcccgta tcgtagttat ctacacgacg 7140

gggagtcagg caactatgga tgaacgaaat agacagatcg ctgagatagg tgcctcactg 7200

attaagcatt ggtaactgtc agaccaagtt tactcatata tactttagat tgatttaaaa 7260

cttcattttt aatttaaaag gatctaggtg aagatccttt ttgataatct catgaccaaa 7320

atcccttaac gtgagttttc gttccactga gcgtcagacc ccgtagaaaa gatcaaagga 7380

tcttcttgag atcctttttt tctgcgcgta atctgctgct tgcaaacaaa aaaaccaccg 7440

ctaccagcgg tggtttgttt gccggatcaa gagctaccaa ctctttttcc gaaggtaact 7500

ggcttcagca gagcgcagat accaaatact gtccttctag tgtagccgta gttaggccac 7560

cacttcaaga actctgtagc accgcctaca tacctcgctc tgctaatcct gttaccagtg 7620

gctgctgcca gtggcgataa gtcgtgtctt accgggttgg actcaagacg atagttaccg 7680

gataaggcgc agcggtcggg ctgaacgggg ggttcgtgca cacagcccag cttggagcga 7740

acgacctaca ccgaactgag atacctacag cgtgagctat gagaaagcgc cacgcttccc 7800

gaagggagaa aggcggacag gtatccggta agcggcaggg tcggaacagg agagcgcacg 7860

agggagcttc cagggggaaa cgcctggtat ctttatagtc ctgtcgggtt tcgccacctc 7920

tgacttgagc gtcgattttt gtgatgctcg tcaggggggc ggagcctatg gaaaaacgcc 7980

agcaacgcgg cctttttacg gttcctggcc ttttgctggc cttgaagctg tccctgatgg 8040

tcgtcatcta cctgcctgga cagcatggcc tgcaacgcgg gcatcccgat gccgccggaa 8100

gcgagaagaa tcataatggg gaaggccatc cagcctcgcg tcgcgaacgc cagcaagacg 8160

tagcccagcg cgtcggcccc gagatgcgcc gcgtgcggct gctggagatg gcggacgcga 8220

tggatatgtt ctgccaaggg ttggtttgcg cattcacagt tctccgcaag aattgattgg 8280

ctccaattct tggagtggtg aatccgttag cgaggtgccg ccctgcttca tccccgtggc 8340

ccgttgctcg cgtttgctgg cggtgtcccc ggaagaaata tatttgcatg tctttagttc 8400

tatgatgaca caaaccccgc ccagcgtctt gtcattggcg aattcgaaca cgcagatgca 8460

gtcggggcgg cgcggtccga ggtccacttc gcatattaag gtgacgcgtg tggcctcgaa 8520

caccgagcga ccctgcagcg acccgcttaa cagcgtcaac agcgtgccgc agatcccggg 8580

gggcaatgag atatgaaaaa gcctgaactc accgcgacgt ctgtcgagaa gtttctgatc 8640

gaaaagttcg acagcgtctc cgacctgatg cagctctcgg agggcgaaga atctcgtgct 8700

ttcagcttcg atgtaggagg gcgtggatat gtcctgcggg taaatagctg cgccgatggt 8760

ttctacaaag atcgttatgt ttatcggcac tttgcatcgg ccgcgctccc gattccggaa 8820

gtgcttgaca ttggggaatt cagcgagagc ctgacctatt gcatctcccg ccgtgcacag 8880

ggtgtcacgt tgcaagacct gcctgaaacc gaactgcccg ctgttctgca gccggtcgcg 8940

gaggccatgg atgcgatcgc tgcggccgat cttagccaga cgagcgggtt cggcccattc 9000

ggaccgcaag gaatcggtca atacactaca tggcgtgatt tcatatgcgc gattgctgat 9060

ccccatgtgt atcactggca aactgtgatg gacgacaccg tcagtgcgtc cgtcgcgcag 9120

gctctcgatg agctgatgct ttgggccgag gactgccccg aagtccggca cctcgtgcac 9180

gcggatttcg gctccaacaa tgtcctgacg gacaatggcc gcataacagc ggtcattgac 9240

tggagcgagg cgatgttcgg ggattcccaa tacgaggtcg ccaacatctt cttctggagg 9300

ccgtggttgg cttgtatgga gcagcagacg cgctacttcg agcggaggca tccggagctt 9360

gcaggatcgc cgcggctccg ggcgtatatg ctccgcattg gtcttgacca actctatcag 9420

agcttggttg acggcaattt cgatgatgca gcttgggcgc agggtcgatg cgacgcaatc 9480

gtccgatccg gagccgggac tgtcgggcgt acacaaatcg cccgcagaag cgcggccgtc 9540

tggaccgatg gctgtgtaga agtactcgcc gatagtggaa accgacgccc cagcactcgt 9600

ccggatcggg agatggggga ggctaactga aacacggaag gagacaatac cggaaggaac 9660

ccgcgctatg acggcaataa aaagacagaa taaaacgcac gggtgttggg tcgtttgttc 9720

ataaacgcgg ggttcggtcc cagggctggc actctgtcga taccccaccg agaccccatt 9780

ggggccaata cgcccgcgtt tcttcctttt ccccacccca ccccccaagt tcgggtgaag 9840

gcccagggct cgcagccaac gtcggggcgg caggccctgc catagccact ggccccgtgg 9900

gttagggacg gggtccccca tggggaatgg tttatggttc gtgggggtta ttattttggg 9960

cgttgcgtgg ggtcaggtcc acgactggac tgagcagaca gacccatggt ttttggatgg 10020

cctgggcatg gaccgcatgt actggcgcga cacgaacacc gggcgtctgt ggctgccaaa 10080

cacccccgac ccccaaaaac caccgcgcgg atttctggcg tgccaagcta gtcgaccaat 10140

tctcatgttt gacagcttat catcgcagat ccgggcaacg ttgttgccat tgctgcaggc 10200

gcagaactgg taggtatgga agatctatac attgaatcaa tattggcaat tagccatatt 10260

agtcattggt tatatagcat aaatcaatat tggctattgg ccattgcata cgttgtatct 10320

atatcataat atgtacattt atattggctc atgtccaata tgaccgccat gttgacattg 10380

attattgact agttattaat agtaatcaat tacggggtca ttagttcata gcccatatat 10440

ggagttccgc gttacataac ttacggtaaa tggcccgcct ggctgaccgc ccaacgaccc 10500

ccgcccattg acgtcaataa tgacgtatgt tcccatagta acgccaatag ggactttcca 10560

ttgacgtcaa tgggtggagt atttacggta aactgcccac ttggcagtac atcaagtgta 10620

tcatatgcca agtccgcccc ctattgacgt caatgacggt aaatggcccg cctggcatta 10680

tgcccagtac atgaccttac gggactttcc tacttggcag tacatctacg tattagtcat 10740

cgctattacc atggtgatgc ggttttggca gtacaccaat gggcgtggat agcggtttga 10800

ctcacgggga tttccaagtc tccaccccat tgacgtcaat gggagtttgt tttggcacca 10860

aaatcaacgg gactttccaa aatgtcgtaa taaccccgcc ccgttgacgc aaatgggcgg 10920

taggcgtgta cggtgggagg tctatataag cagagctcgt ttagtgaacc gtcagatctc 10980

tagaagctgg gtac 10994

<210> 9

<211>  12539
<212>  DNA
<213>  artificial

<220>
<223>  plasmid vector encoding Fc-tag IL-12 fusion construct

<400>  9
gtacctcgca gcaaagcaag atgtgtcctc agaagctaac catctcctgg tttgccatcg      60

ttttgctggt gtctccactc atggccatgt gggagctgga gaaagacgtt tatgttgtag     120

aggtggactg gactcccgat gcccctggag aaacagtgaa cctcacctgt gacacgcctg     180

aagaagatga catcacctgg acctcagacc agagacatgg agtcataggc tctggaaaga     240

ccctgaccat cactgtcaaa gagtttctag atgctggcca gtacacctgc cacaaaggag     300

gcgagactct gagccactca catctgctgc tccacaagaa ggaaaatgga atttggtcca     360

ctgaaatttt aaaaaatttc aaaaacaaga ctttcctgaa gtgtgaagca ccaaattact     420

ccggacggtt cacgtgctca tggctggtgc aaagaaacat ggacttgaag ttcaacatca     480

agagcagtag cagttcccct gactctcggg cagtgacatg tggaatggcg tctctgtctg     540

cagagaaggt cacactggac caaagggact atgagaagta ttcagtgtcc tgccaggagg     600

atgtcacctg cccaactgcc gaggagaccc tgcccattga actggcgttg gaagcacggc     660

agcagaataa atatgagaac tacagcacca gcttcttcat cagggacatc atcaaaccag     720

acccgcccaa gaacttgcag atgaagcctt tgaagaactc acaggtggag gtcagctggg     780

agtaccctga ctcctggagc actccccatt cctacttctc cctcaagttc tttgttcgaa     840

tccagcgcaa gaaagaaaag atgaaggaga cagaggaggg gtgtaaccag aaaggtgcgt     900

tcctcgtaga gaagacatct accgaagtcc aatgcaaagg cgggaatgtc tgcgtgcaag     960

ctcaggatcg ctattacaat tcctcgtgca gcaagtgggc atgtgttccc tgcagggtcc    1020

gatccggagg cggtggctcg ggcggtggtg ggtcgggtgg cggcggatcc agggtcattc    1080

cagtctctgg acctgccagg tgtcttagcc agtcccgaaa cctgctgaag accacagatg    1140

acatggtgaa gacggccaga gaaaaactga aacattattc ctgcactgct gaagacatcg    1200

atcatgaaga catcacacgg gaccaaacca gcacattgaa gacctgttta ccactggaac    1260

tacacaagaa cgagagttgc ctggctacta gagagacttc ttccacaaca agagggagct    1320

gcctgccccc acagaagacg tctttgatga tgaccctgtg ccttggtagc atctatgagg    1380

acttgaagat gtaccagaca gagttccagg ccatcaacgc agcacttcag aatcacaacc    1440

atcagcagat cattctagac aagggcatgc tggtggccat cgatgagctg atgcagtctc    1500

tgaatcataa tggcgagact ctgcgccaga aacctcctgt gggagaagca gacccttaca    1560

gagtgaaaat gaagctctgc atcctgcttc acgccttcag cacccgcgtc gtgaccatca    1620

acagggtgat gggctatctg agctccgcct tgatcaagag aatcgagcct agaataccca    1680

agcccagtac ccccccaggt tcttcatgcc cacctggtaa catcttgggt ggaccatccg 1740

tcttcatctt ccccccaaag cccaaggatg cactcatgat ctccctaacc cccaaggtta 1800

cgtgtgtggt ggtggatgtg agcgaggatg acccagatgt ccatgtcagc tggtttgtgg 1860

acaacaaaga agtacacaca gcctggacgc agccccgtga agctcagtac aacagtacct 1920

tccgagtggt cagtgccctc cccatccagc accaggactg gatgaggggc aaggagttca 1980

aatgcaaggt caacaacaaa gccctcccag cccccatcga gagaaccatc tcaaaaccca 2040

aaggaagagc ccagacacct caagtataca ccataccccc acctcgtgaa caaatgtcca 2100

agaagaaggt tagtctgacc tgcctggtca ccaacttctt ctctgaagcc atcagtgtgg 2160

agtgggaaag gaacggagaa ctggagcagg attacaagaa cactccaccc atcctggact 2220

cggatgggac ctacttcctc tacagcaagc tcactgtgga tacagacagt tggttgcaag 2280

gagaaatttt tacctgctcc gtggtgcatg aggctctcca taaccaccac acacagaaga 2340

acctgtctcg ctcccctggt aaatgagaac agcatctagc ggccgctcga ggccggcaag 2400

gccggatcca gacatgataa gatacattga tgagtttgga caaaccacaa ctagaatgca 2460

gtgaaaaaaa tgctttattt gtgaaatttg tgatgctatt gctttatttg taaccattat 2520

aagctgcaat aaacaagtta acaacaacaa ttgcattcat tttatgtttc aggttcaggg 2580

ggaggtgtgg gaggtttttt aaagcaagta aaacctctac aaatgtggta tggctgatta 2640

tgatccggct gcctcgcgcg tttcggtgat gacggtgaaa acctctgaca catgcagctc 2700

ccggagacgg tcacagcttg tctgtaagcg gatgccggga gcagacaagc ccgtcaggcg 2760

tcagcgggtg ttggcgggtg tcggggcgca gccatgaggt cgactctaga ggatcgatgc 2820

cccgccccgg acgaactaaa cctgactacg acatctctgc cccttcttcg cggggcagtg 2880

catgtaatcc cttcagttgg ttggtacaac ttgccaactg ggccctgttc cacatgtgac 2940

acgggggggg accaaacaca aaggggttct ctgactgtag ttgacatcct tataaatgga 3000

tgtgcacatt tgccaacact gagtggcttt catcctggag cagactttgc agtctgtgga 3060

ctgcaacaca acattgcctt tatgtgtaac tcttggctga agctcttaca ccaatgctgg 3120

gggacatgta cctcccaggg gcccaggaag actacgggag gctacaccaa cgtcaatcag 3180

aggggcctgt gtagctaccg ataagcggac cctcaagagg gcattagcaa tagtgtttat 3240

aaggccccct tgttaaccct aaacgggtag catatgcttc ccgggtagta gtatatacta 3300

tccagactaa ccctaattca atagcatatg ttacccaacg ggaagcatat gctatcgaat 3360

tagggttagt aaaagggtcc taaggaacag cgatatctcc caccccatga gctgtcacgg 3420

ttttatttac atggggtcag gattccacga gggtagtgaa ccattttagt cacaagggca 3480

gtggctgaag atcaaggagc gggcagtgaa ctctcctgaa tcttcgcctg cttcttcatt 3540

ctccttcgtt tagctaatag aataactgct gagttgtgaa cagtaaggtg tatgtgaggt 3600
gctcgaaaac aaggtttcag gtgacgcccc cagaataaaa tttggacggg gggttcagtg 3660
gtggcattgt gctatgacac caatataacc ctcacaaacc ccttgggcaa taaatactag 3720
tgtaggaatg aaacattctg aatatcttta acaatagaaa tccatggggt ggggacaagc 3780
cgtaaagact ggatgtccat ctcacacgaa tttatggcta tgggcaacac ataatcctag 3840
tgcaatatga tactggggtt attaagatgt gtcccaggca gggaccaaga caggtgaacc 3900
atgttgttac actctatttg taacaagggg aaagagagtg gacgccgaca gcagcggact 3960
ccactggttg tctctaacac ccccgaaaat taaacggggc tccacgccaa tggggcccat 4020
aaacaaagac aagtggccac tctttttttt gaaattgtgg agtgggggca cgcgtcagcc 4080
cccacacgcc gccctgcggt tttggactgt aaaataaggg tgtaataact tggctgattg 4140
taaccccgct aaccactgcg gtcaaaccac ttgcccacaa aaccactaat ggcaccccgg 4200
ggaatacctg cataagtagg tgggcgggcc aagatagggg cgcgattgct gcgatctgga 4260
ggacaaatta cacacacttg cgcctgagcg ccaagcacag ggttgttggt cctcatattc 4320
acgaggtcgc tgagagcacg gtgggctaat gttgccatgg gtagcatata ctacccaaat 4380
atctggatag catatgctat cctaatctat atctgggtag cataggctat cctaatctat 4440
atctgggtag catatgctat cctaatctat atctgggtag tatatgctat cctaatttat 4500
atctgggtag cataggctat cctaatctat atctgggtag catatgctat cctaatctat 4560
atctgggtag tatatgctat cctaatctgt atccgggtag catatgctat cctaatagag 4620
attagggtag tatatgctat cctaatttat atctgggtag catatactac ccaaatatct 4680
ggatagcata tgctatccta atctatatct gggtagcata tgctatccta atctatatct 4740
gggtagcata ggctatccta atctatatct gggtagcata tgctatccta atctatatct 4800
gggtagtata tgctatccta atttatatct gggtagcata ggctatccta atctatatct 4860
gggtagcata tgctatccta atctatatct gggtagtata tgctatccta atctgtatcc 4920
gggtagcata tgctatcctc atgcatatac agtcagcata tgatacccag tagtagagtg 4980
ggagtgctat cctttgcata tgccgccacc tcccaagggg gcgtgaattt tcgctgcttg 5040
tccttttcct gctggttgct cccattctta ggtgaattta aggaggccag gctaaagccg 5100
tcgcatgtct gattgctcac caggtaaatg tcgctaatgt tttccaacgc gagaaggtgt 5160
tgagcgcgga gctgagtgac gtgacaacat gggtatgccc aattgcccca tgttgggagg 5220
acgaaaatgg tgacaagaca gatggccaga aatacaccaa cagcacgcat gatgtctact 5280
ggggatttat tctttagtgc gggggaatac acggctttta atacgattga gggcgtctcc 5340
taacaagtta catcactcct gcccttcctc accctcatct ccatcacctc cttcatctcc 5400
gtcatctccg tcatcaccct ccgcggcagc cccttccacc ataggtggaa accagggagg 5460

caaatctact ccatcgtcaa agctgcacac agtcaccctg atattgcagg taggagcggg 5520

ctttgtcata acaaggtcct taatcgcatc cttcaaaacc tcagcaaata tatgagtttg 5580

taaaaagacc atgaaataac agacaatgga ctcccttagc gggccaggtt gtgggccggg 5640

tccaggggcc attccaaagg ggagacgact caatggtgta agacgacatt gtggaatagc 5700

aagggcagtt cctcgcctta ggttgtaaag ggaggtctta ctacctccat atacgaacac 5760

accggcgacc caagttcctt cgtcggtagt cctttctacg tgactcctag ccaggagagc 5820

tcttaaacct tctgcaatgt tctcaaattt cgggttggaa cctccttgac cacgatgctt 5880

tccaaaccac cctccttttt tgcgcctgcc tccatcaccc tgaccccggg gtccagtgct 5940

tgggccttct cctgggtcat ctgcggggcc ctgctctatc gctcccgggg gcacgtcagg 6000

ctcaccatct gggccacctt cttggtggta ttcaaaataa tcggcttccc ctacagggtg 6060

gaaaaatggc cttctacctg gagggggcct gcgcggtgga gacccggatg atgatgactg 6120

actactggga ctcctgggcc tcttttctcc acgtccacga cctctccccc tggctctttc 6180

acgacttccc cccctggctc tttcacgtcc tctaccccgg cggcctccac tacctcctcg 6240

accccggcct ccactacctc ctcgaccccg gcctccactg cctcctcgac cccggcctcc 6300

acctcctgct cctgcccctc ctgctcctgc ccctcctcct gctcctgccc ctcctgcccc 6360

tcctgctcct gcccctcctg cccctcctgc tcctgcccct cctgcccctc ctgctcctgc 6420

ccctcctgcc cctcctcctg ctcctgcccc tcctgcccct cctcctgctc ctgcccctcc 6480

tgcccctcct gctcctgccc ctcctgcccc tcctgctcct gcccctcctg cccctcctgc 6540

tcctgcccct cctgctcctg cccctcctgc tcctgcccct cctgctcctg cccctcctgc 6600

ccctcctgcc cctcctcctg ctcctgcccc tcctgctcct gcccctcctg cccctcctgc 6660

ccctcctgct cctgcccctc ctcctgctcc tgcccctcct gcccctcctg cccctcctcc 6720

tgctcctgcc cctcctgccc ctcctcctgc tcctgcccct cctcctgctc ctgcccctcc 6780

tgcccctcct gcccctcctc ctgctcctgc ccctcctgcc cctcctcctg ctcctgcccc 6840

tcctcctgct cctgcccctc ctgcccctcc tgcccctcct cctgctcctg cccctcctcc 6900

tgctcctgcc cctcctgccc ctcctgcccc tcctgcccct cctcctgctc ctgcccctcc 6960

tcctgctcct gcccctcctg ctcctgcccc tcccgctcct gctcctgctc ctgttccacc 7020

gtgggtccct ttgcagccaa tgcaacttgg acgtttttgg ggtctccgga caccatctct 7080

atgtcttggc cctgatcctg agccgcccgg ggctcctggt cttccgcctc ctcgtcctcg 7140

tcctcttccc cgtcctcgtc catggttatc accccctctt ctttgaggtc cactgccgcc 7200

ggagccttct ggtccagatg tgtctccctt ctctcctagg ccatttccag gtcctgtacc 7260

tggcccctcg tcagacatga ttcacactaa aagagatcaa tagacatctt tattagacga 7320

cgctcagtga atacagggag tgcagactcc tgccccctcc aacagccccc ccaccctcat 7380

ccccttcatg gtcgctgtca gacagatcca ggtctgaaaa ttccccatcc tccgaaccat 7440

cctcgtcctc atcaccaatt actcgcagcc cggaaaactc ccgctgaaca tcctcaagat 7500

ttgcgtcctg agcctcaagc caggcctcaa attcctcgtc cccctttttg ctggacggta 7560

gggatgggga ttctcgggac ccctcctctt cctcttcaag gtcaccagac agagatgcta 7620

ctggggcaac ggaagaaaag ctgggtgcgg cctgtgagga tcagcttatc gatgataagc 7680

tgtcaaacat gagaattctt gaagacgaaa gggcctcgtg atacgcctat ttttataggt 7740

taatgtcatg ataataatgg tttcttagac gtcaggtggc acttttcggg gaaatgtgcg 7800

cggaacccct atttgtttat ttttctaaat acattcaaat atgtatccgc tcatgagaca 7860

ataaccctga taaatgcttc aataatattg aaaaaggaag agtatgagta ttcaacattt 7920

ccgtgtcgcc cttattccct tttttgcggc attttgcctt cctgtttttg ctcacccaga 7980

aacgctggtg aaagtaaaag atgctgaaga tcagttgggt gcacgagtgg gttacatcga 8040

actggatctc aacagcggta agatccttga gagttttcgc cccgaagaac gttttccaat 8100

gatgagcact tttaaagttc tgctatgtgg cgcggtatta tcccgtgttg acgccgggca 8160

agagcaactc ggtcgccgca tacactattc tcagaatgac ttggttgagt actcaccagt 8220

cacagaaaag catcttacgg atggcatgac agtaagagaa ttatgcagtg ctgccataac 8280

catgagtgat aacactgcgg ccaacttact tctgacaacg atcggaggac cgaaggagct 8340

aaccgctttt ttgcacaaca tgggggatca tgtaactcgc cttgatcgtt gggaaccgga 8400

gctgaatgaa gccataccaa acgacgagcg tgacaccacg atgcctgcag caatggcaac 8460

aacgttgcgc aaactattaa ctggcgaact acttactcta gcttcccggc aacaattaat 8520

agactggatg gaggcggata aagttgcagg accacttctg cgctcggccc ttccggctgg 8580

ctggtttatt gctgataaat ctggagccgg tgagcgtggg tctcgcggta tcattgcagc 8640

actggggcca gatggtaagc cctcccgtat cgtagttatc tacacgacgg ggagtcaggc 8700

aactatggat gaacgaaata gacagatcgc tgagataggt gcctcactga ttaagcattg 8760

gtaactgtca gaccaagttt actcatatat actttagatt gatttaaaac ttcattttta 8820

atttaaaagg atctaggtga agatcctttt tgataatctc atgaccaaaa tcccttaacg 8880

tgagttttcg ttccactgag cgtcagaccc cgtagaaaag atcaaaggat cttcttgaga 8940

tccttttttt ctgcgcgtaa tctgctgctt gcaaacaaaa aaaccaccgc taccagcggt 9000

ggtttgtttg ccggatcaag agctaccaac tctttttccg aaggtaactg gcttcagcag 9060

agcgcagata ccaaatactg tccttctagt gtagccgtag ttaggccacc acttcaagaa 9120

ctctgtagca ccgcctacat acctcgctct gctaatcctg ttaccagtgg ctgctgccag 9180

tggcgataag tcgtgtctta ccgggttgga ctcaagacga tagttaccgg ataaggcgca 9240

gcggtcgggc tgaacggggg gttcgtgcac acagcccagc ttggagcgaa cgacctacac 9300

cgaactgaga tacctacagc gtgagctatg agaaagcgcc acgcttcccg aagggagaaa 9360

ggcggacagg tatccggtaa gcggcagggt cggaacagga gagcgcacga gggagcttcc 9420

agggggaaac gcctggtatc tttatagtcc tgtcgggttt cgccacctct gacttgagcg 9480

tcgatttttg tgatgctcgt caggggggcg gagcctatgg aaaaacgcca gcaacgcggc 9540

ctttttacgg ttcctggcct tttgctggcc ttgaagctgt ccctgatggt cgtcatctac 9600

ctgcctggac agcatggcct gcaacgcggg catcccgatg ccgccggaag cgagaagaat 9660

cataatgggg aaggccatcc agcctcgcgt cgcgaacgcc agcaagacgt agcccagcgc 9720

gtcggccccg agatgcgccg cgtgcggctg ctggagatgg cggacgcgat ggatatgttc 9780

tgccaagggt tggtttgcgc attcacagtt ctccgcaaga attgattggc tccaattctt 9840

ggagtggtga atccgttagc gaggtgccgc cctgcttcat ccccgtggcc cgttgctcgc 9900

gtttgctggc ggtgtccccg gaagaaatat atttgcatgt ctttagttct atgatgacac 9960

aaaccccgcc cagcgtcttg tcattggcga attcgaacac gcagatgcag tcggggcggc 10020

gcggtccgag gtccacttcg catattaagg tgacgcgtgt ggcctcgaac accgagcgac 10080

cctgcagcga cccgcttaac agcgtcaaca gcgtgccgca gatcccgggg ggcaatgaga 10140

tatgaaaaag cctgaactca ccgcgacgtc tgtcgagaag tttctgatcg aaaagttcga 10200

cagcgtctcc gacctgatgc agctctcgga gggcgaagaa tctcgtgctt tcagcttcga 10260

tgtaggaggg cgtggatatg tcctgcgggt aaatagctgc gccgatggtt tctacaaaga 10320

tcgttatgtt tatcggcact ttgcatcggc cgcgctcccg attccggaag tgcttgacat 10380

tggggaattc agcgagagcc tgacctattg catctcccgc cgtgcacagg gtgtcacgtt 10440

gcaagacctg cctgaaaccg aactgcccgc tgttctgcag ccggtcgcgg aggccatgga 10500

tgcgatcgct gcggccgatc ttagccagac gagcgggttc ggcccattcg gaccgcaagg 10560

aatcggtcaa tacactacat ggcgtgattt catatgcgcg attgctgatc cccatgtgta 10620

tcactggcaa actgtgatgg acgacaccgt cagtgcgtcc gtcgcgcagg ctctcgatga 10680

gctgatgctt tgggccgagg actgccccga agtccggcac ctcgtgcacg cggatttcgg 10740

ctccaacaat gtcctgacgg acaatggccg cataacagcg gtcattgact ggagcgaggc 10800

gatgttcggg gattcccaat acgaggtcgc caacatcttc ttctggaggc cgtggttggc 10860

ttgtatggag cagcagacgc gctacttcga gcggaggcat ccggagcttg caggatcgcc 10920

gcggctccgg gcgtatatgc tccgcattgg tcttgaccaa ctctatcaga gcttggttga 10980

cggcaatttc gatgatgcag cttgggcgca gggtcgatgc gacgcaatcg tccgatccgg 11040

agccgggact gtcgggcgta cacaaatcgc ccgcagaagc gcggccgtct ggaccgatgg 11100

ctgtgtagaa gtactcgccg atagtggaaa ccgacgcccc agcactcgtc cggatcggga 11160

gatgggggag gctaactgaa acacggaagg agacaatacc ggaaggaacc cgcgctatga 11220

cggcaataaa aagacagaat aaaacgcacg ggtgttgggt cgtttgttca taaacgcggg 11280

gttcggtccc agggctggca ctctgtcgat accccaccga gaccccattg gggccaatac 11340

gcccgcgttt cttccttttc cccaccccac cccccaagtt cgggtgaagg cccagggctc 11400

gcagccaacg tcggggcggc aggccctgcc atagccactg gccccgtggg ttagggacgg 11460

ggtcccccat ggggaatggt ttatggttcg tgggggttat tattttgggc gttgcgtggg 11520

gtcaggtcca cgactggact gagcagacag acccatggtt tttggatggc ctgggcatgg 11580

accgcatgta ctggcgcgac acgaacaccg ggcgtctgtg gctgccaaac acccccgacc 11640

cccaaaaacc accgcgcgga tttctggcgt gccaagctag tcgaccaatt ctcatgtttg 11700

acagcttatc atcgcagatc cgggcaacgt tgttgccatt gctgcaggcg cagaactggt 11760

aggtatggaa gatctataca ttgaatcaat attggcaatt agccatatta gtcattggtt 11820

atatagcata aatcaatatt ggctattggc cattgcatac gttgtatcta tatcataata 11880

tgtacattta tattggctca tgtccaatat gaccgccatg ttgacattga ttattgacta 11940

gttattaata gtaatcaatt acggggtcat tagttcatag cccatatatg gagttccgcg 12000

ttacataact tacggtaaat ggcccgcctg gctgaccgcc caacgacccc cgcccattga 12060

cgtcaataat gacgtatgtt cccatagtaa cgccaatagg gactttccat tgacgtcaat 12120

gggtggagta tttacggtaa actgcccact tggcagtaca tcaagtgtat catatgccaa 12180

gtccgccccc tattgacgtc aatgacggta aatggcccgc ctggcattat gcccagtaca 12240

tgaccttacg ggactttcct acttggcagt acatctacgt attagtcatc gctattacca 12300

tggtgatgcg gttttggcag tacaccaatg ggcgtggata gcggtttgac tcacggggat 12360

ttccaagtct ccaccccatt gacgtcaatg ggagtttgtt ttggcaccaa aatcaacggg 12420

actttccaaa atgtcgtaat aaccccgccc cgttgacgca aatgggcggt aggcgtgtac 12480

ggtgggaggt ctatataagc agagctcgtt tagtgaaccg tcagatctct agaagctgg 12539

## Description

The present invention relates to compositions and methods for treating cancer, in particular, to immunotherapy of malignant neoplastic disease such as glioma, by administering an effective dose of a polypeptide with IL-12 biological activity and a non-agonist ligand of a T-cell downregulator, particularly a non-agonist ligand to CTLA-4 and/or to Programmed Death 1 (PD-1).

Glioblastoma multiforme (GBM) is the most malignant astrocytic tumour. GBM exhibits an invasive and destructive growth pattern; it is the most common and most aggressive malignant primary brain tumour in humans, accounting for 20 % of all intracranial tumours. In most European countries and North America, GBM incidence is in the range of 3-3.5 new cases per 100'000 population per year. The clinical history of the disease is usually short (less than 3 months in more than 50% of cases) and patients diagnosed with GBM show a median survival of 14-18 months despite aggressive surgery, radiation, and chemotherapy. The ability of gliomas to withstand conventional treatment regimens is one of the greatest challenges of modern neuro-oncology.

Interleukin (IL)-12 is the prototype of a group of heterodimeric cytokines with predominantly inflammatory properties. IL-12 polarizes naive helper T-cells to adopt a TH1 phenotype and stimulates cytotoxic T and NK-cells. IL-12 binds to the IL-12 receptor (IL-12R), which is a heterodimeric receptor formed by IL-12R-β1 and IL-12R-β2.The receptor complex is primarily expressed by T cells, but also other lymphocyte subpopulations have been found to be responsive to IL-12.

The therapeutic application of IL-12 in various tumour entities has been suggested. Clinical trials in cancer patients, however, had to be halted since systemic application evoked serious adverse events at effective doses, including fatalities. While research in recent years has mainly focused on various administration routes of IL-12, there remain open questions on the exact mechanisms by which IL-12 exerts its tumour-suppressive properties.

CTLA-4 and PD-1 are both members of the extended CD28/CTLA-4 family of T cell regulators. PD-1 is expressed on the surface of activated T cells, B cells and macrophages. PD-1(CD279; Uniprot Q15116) has two ligands, PD-L1 (B7-H1, CD274) and PD-L2 (B7-DC, CD273), which are members of the B7 family.

CTLA-4 (Uniprot ID No P16410) is expressed on the surface of T helper cells and transmits an inhibitory signal to T lymphocytes. CTLA-4 and CD28 bind to CD80 (B7-1) and CD86 (B7-2) on antigen-presenting cells. CTLA-4 transmits an inhibitory signal to T cells, whereas CD28 transmits a stimulatory signal. Systemic anti-CTLA-4 treatment has been approved for clinical use and demonstrates clinical benefit. It is being further tested for various other solid cancers (Hodi et al., N Engl J Med 363, 711-723 (2010); Graziani et al., Pharmacol Res (2012) Jan;65(1):9-22). A commercial antibody against CTLA-4 is available under the generic name ipilimumab (marketed as *Yervoy*).

Various anti-PD-L1 antibodies (e.g. MDX-1105/BMS-936559) and anti-PD-1 antibodies are currently undergoing clinical trials (e.g. MDX-1106/BMS-936558/ONO-4538 or MK-3475/SCH 900475 or AMP-224).

The glycoprotein immunoglobulin G (IgG) is a major effector molecule of the humoral immune response in man. There are four distinct subgroups of human IgG designated IgG1, IgG2, IgG3 and IgG4. The four subclasses show more than 95% homology in the amino acid sequences of the constant domains of the heavy chains, but differ with respect to structure and flexibility of the hinge region, especially in the number of inter-heavy chain disulfide bonds in this domain. The structural differences between the IgG subclasses are also reflected in their susceptibility to proteolytic enzymes, such as papain, plasmin, trypsin and pepsin.

Only one isoform of human IgG4 is known. In contrast to human IgG1, IgG2 and IgG3, human IgG4 does not activate complement. Furthermore, IgG4 is less susceptible to proteolytic enzymes compared to IgG2 and IgG3.

The problem underlying the present invention is the provision of improved means and methods for treating solid cancer, in particular glioma.

In the course of a study focused on the clinical therapeutic potential of IL-12 in advanced-stage GBM in a relevant rodent model, it was surprisingly found that the combination of IL-12 with a blockade of co-inhibitory signals with anti-CTLA-4 antibody leads to almost complete tumour eradication and cure even at advanced disease stages. The combination of IL-12 with a blockade of co-inhibitory signals with anti-PD-1 antibody as well leads to tumour regression.

According to a first aspect of the invention, a combination medicament is provided for use in the therapy of solid tumours, particular brain tumours, particularly glioma, which comprises
- an IL-12 polypeptide and
- a T cell inhibition blocker agent selected from
   - a non-agonist CTLA-4 ligand and
   - a non-agonist PD-1 or PD-L1 or PD-L2 ligand.

In the context of the present invention, an IL-12 polypeptide is a polypeptide having an amino acid sequence comprising the sequence of p35 (Uniprot ID 29459, SEQ ID 05) or a functional homologue thereof, and comprising the sequence of p40 (Uniprot ID29460, SEQ ID 06) or a functional homologue thereof. In one embodiment, the IL-12 polypeptide has an amino acid sequence comprising both p35 and p40 sequences or homologues thereof as part of the same continuous amino acid chain. In another embodiment, the IL-12 polypeptide comprises two distinct amino acid chains, one comprising the p35 sequence and another one comprising the p40 sequence. The terminology "IL-12 polypeptide" does not preclude the presence of non-IL-12 sequences, for example immunoglobulin sequences and fragments thereof, fused to the IL-12 sequences described herein.

The IL-12 polypeptide has a biological activity of IL-12. A biological activity of IL-12 in the context of the present invention is the stimulation of NK or T cells by said IL-12 polypeptide, most prominently the stimulation of T effector cells acting through perforin.

In one embodiment of the combination medicament, said IL-12 polypeptide comprises a polypeptide sequence at least 95%, 96%, 97%, 98% or 99% identical to the sequence of human p35 (SEQ ID 05), and a polypeptide sequence at least 95%, 96%, 97%, 98% or 99% identical to the sequence of human p40 (SEQ ID 06).

Identity in the context of the present invention is a single quantitative parameter representing the result of a sequence comparison position by position. Methods of sequence comparison are known in the art; the BLAST algorithm available publicly is an example.

In one embodiment, said IL-12 polypeptide is a recombinant human IL-12. In one embodiment, said IL-12 polypeptide is a synthetic human IL-12. In one embodiment, said IL-12 polypeptide is a fusion peptide comprising the crystallisable fragment (Fc region) of a human immunoglobulin. According to one embodiment, the IL-12 polypeptide comprises a crystallisable fragment of human immunoglobulin G. A crystallizable fragment in the context of the present invention refers to the second and third constant domain of the IgG molecule. The fragment crystallizable region (Fc region) is the tail region of an immunoglobulin antibody that interacts with cell surface receptors (Fc receptors) and proteins of the complement system. In IgG antibody isotypes, the Fc region is composed of two identical protein fragments, derived from the second and third constant domains of the antibody's two heavy chains.

According to one embodiment, the IL-12 polypeptide comprises a crystallisable fragment of human immunoglobulin G4. According to one embodiment, the IL-12 polypeptide has or comprises the sequence of SEQ ID 01. According to another embodiment, the IL-12 polypeptide comprises a sequence at least 95%, 96%, 97%, 98% or 99% identical to the sequence of SEQ ID 01.

Embodiments wherein IL-12 polypeptide chains are fused to immunoglobulin Fc fragments show different pharmacokinetic behaviour in comparison to the recombinant cytokine, which for some applications may confer a benefit.

In one embodiment, the IL-12 polypeptide component of the combination medicament is provided as a dosage form for local (intratumoural) administration or delivery. Such dosage form for local (intratumoural) administration may be a slow-release form or depot form, from which said IL-12 polypeptide is released over a number of hours to weeks. In one embodiment, the IL-12 polypeptide component of the combination medicament is administered via convection enhanced delivery (CED) or a variation thereof, for example the device shown in US2011137289 (A1) (incorporated herein by reference).

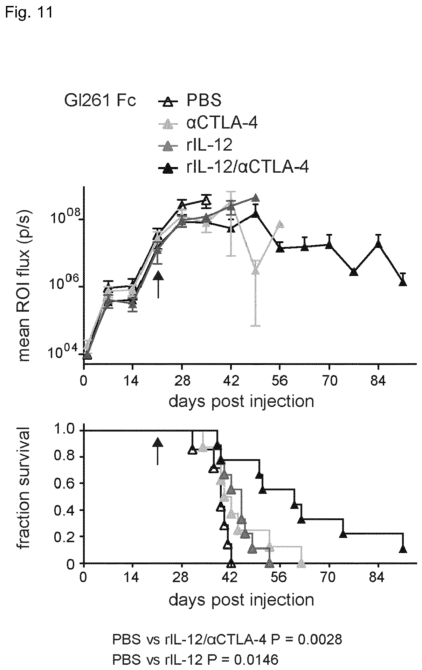

In one embodiment, the IL-12 polypeptide is administered systemically together with systemic CTLA-4/PD-1/PD-L1/PD-L2 blockade. Heterodimeric recombinant IL-12 (peprotech) applied systemically together with systemic CTLA-4 blockade (i.p.) achieved a significant improvement in survival in comparison to either agent administered by itself (see Fig. 11).

In the context of the present invention, a non-agonist CTLA-4 ligand is a molecule that binds selectively to CTLA-4 under conditions prevailing in peripheral blood, without triggering the biological effect of CTLA-4 interaction with any of the physiological ligands of CTLA-4, particularly CD80 and/or CD86.

In the context of the present invention, a non-agonist PD-1 ligand is a molecule that binds selectively to PD-1 under conditions prevailing in peripheral blood, without triggering the biological effect of PD-1 interaction with any of the physiological ligands of PD-1, particularly PD-L1 or PD-L2. A non-agonist PD-L1 (PD-L2) ligand is a molecule that binds selectively to to PD-L1 (or to PD-L2) under conditions prevailing in peripheral blood, without triggering the biological effect of PD-L1 (PD-L2) interaction with any of its physiological ligands, particularly PD-1.

In some embodiments, said non-agonist CTLA-4 ligand is a polypeptide binding to CTLA-4. In some embodiments, said non-agonist PD-1 ligand is a polypeptide binding to PD-1.

A non-agonist CTLA-4 ligand in the sense of the invention refers to a molecule that is capable of binding to CTLA-4 with a dissociation constant of at least 10⁻⁷ M⁻¹, 10⁻⁸ M⁻¹ or 10⁻⁹ M⁻¹ and which inhibits the biological activity of its respective target. A a non-agonist PD-1 ligand or a non-agonist PD-L1 (PD-L2) ligand in the sense of the invention refers to a molecule that is capable of binding to PD-1 (PD-L1, PD-L2) with a dissociation constant of at least 10⁻⁷ M⁻¹, 10⁻⁸ M⁻¹ or 10⁻⁹ M⁻¹ and which inhibits the biological activity of its respective target.

A non-agonist polypeptide ligand may be an antibody, an antibody fragment, an antibody-like molecule or an oligopeptide, any of which binds to and thereby inhibits CTLA-4, PD-1 or PD-L1 (PD-L2), respectively.

An antibody fragment may be a Fab domain or an Fv domain of an antibody, or a single-chain antibody fragment, which is a fusion protein consisting of the variable regions of light and heavy chains of an antibody connected by a peptide linker. The inhibitor may also be a single domain antibody, consisting of an isolated variable domain from a heavy or light chain. Additionally, an antibody may also be a heavy-chain antibody consisting of only heavy chains such as antibodies found in camelids. An antibody-like molecule may be a repeat protein, such as a designed ankyrin repeat protein (Molecular Partners, Zürich).

An oligopeptide according to the above aspect of the invention may be a peptide derived from the recognition site of a physiological ligand of CTLA-4, PD-1 or PD-L1 or PD-L2. Such oligopeptide ligand competes with the physiological ligand for binding to CTLA-4, PD-1 or PD-L1 or PD-L2, respectively.

Particularly, a non-agonist CTLA-4 ligand or non-agonist PD-1 ligand or non-agonist PD-L1 ligand or non-agonist PD-L2 ligand does not lead to attenuated T cell activity when binding to CTLA-4, PD-1, PD-L1 or PD-L2, respectively, on the surface on a T-cell. In certain embodiments, the term "non-agonist CTLA-4 ligand" or "non-agonist PD-1 ligand" covers both antagonists of CTLA-4 or PD-1 and ligands that are neutral vis-à-vis CTLA-4 or PD-1 signalling. In some embodiments, non-agonist CTLA-4 ligands used in the present invention are able, when bound to CTLA-4, to sterically block interaction of CTLA-4 with its binding partners CD80 and/or CD86 and non-agonist PD-1 ligands used in the present invention are able, when bound to PD-1, to sterically block interaction of PD-1 with its binding partners PD-L1 and/or PD-L2.

In one embodiment, said non-agonist CTLA-4 ligand is a gamma immunoglobulin binding to CTLA-4, without triggering the physiological response of CTLA-4 interaction with its binding partners CD80 and/or CD86.

In some embodiments, said non-agonist PD-1 ligand is a gamma immunoglobulin binding to PD-1, without triggering the physiological response of PD-1 interaction with its binding partners PD-L1 and/or PD-L2.

In some embodiments, said non-agonist PD-L1 (PD-L2) ligand is a gamma immunoglobulin binding to PD-L1 (PD-L2), without triggering the physiological response of PD-1 interaction with its binding partners PD-L1 and/or PD-L2.

Non-limiting examples for a CTLA-4 ligand are the clinically approved antibodies tremelimumab (CAS 745013-59-6) and ipilimumab (CAS No. 477202-00-9; Yervoy).

Non-limiting examples for a PD-1 / PD-L1 or PD-L2 ligands are the antibodies MDX-1105/BMS-936559, MDX-1106/BMS-936558/ONO-4538, MK-3475/SCH 900475 or AMP-224 currently undergoing clinical development

The term "gamma immunoglobulin" in this context is intended to encompass both complete immunoglobulin molecules and functional fragments thereof, wherein the function is binding to CTLA-4, PD-1 or PD-L1 (PD-L2) as laid out above.

In one embodiment, the combination therapy comprises two distinct dosage forms, wherein said IL-12 polypeptide is provided as a dosage form for intratumoural delivery or local delivery in the vicinity of the tumour, and said non-agonist CTLA-4 ligand or non-agonist PD-1 ligand is provided as a dosage form for systemic delivery, particularly by intravenous injection. However, said non-agonist CTLA-4 ligand or non-agonist PD-1 ligand may also be locally applied in the same way as the IL-12 polypeptide. According to another embodiment, the IL-12 polypeptide is applied directly to the tumour draining lymph node.

According to another embodiment, the combination therapy comprises a dosage form whereby said IL-12 polypeptide is provided for intracranial delivery, e.g. by injection.

According to another aspect of the invention, a combination medicament is provided as set forth above, for use in a method of therapy of a malignant neoplastic disease, particularly solid cancerous lesions. In one embodiment, the malignant neoplastic disease is glioma. In one embodiment, the malignant neoplastic disease is a secondary brain tumour (brain metastasis of a neoplastic lesion arising outside the brain). In one embodiment, the disease is glioblastoma multiforme. In one embodiment, the malignant neoplastic disease is meningioma. In one embodiment, the malignant neoplastic disease is melanoma. In one embodiment, the malignant neoplastic disease is pancreatic cancer. In one embodiment, the malignant neoplastic disease is lung cancer. In one embodiment, the malignant neoplastic disease is prostate cancer. In one embodiment, the malignant neoplastic disease is bladder cancer.

Cancerous lesions have the propensity to spread into neighbouring tissue as well as distinct locations in the body, depending on their origin. 20-40% of all cancers develop brain metastasis; among those lung, breast and skin (melanoma) cancer are the most common sources of brain metastases (Sofietti et al., J Neurol 249, 1357-1369 (2002)). Similar to primary malignant brain tumours, brain metastases have a poor prognosis despite treatment and are quickly fatal. T-cells are the crucial effector cell population for IL-12 mediated tumor rejection in the brain. IL-12 together with anti-CTLA-4, anti-PD-1, anti-PD-L1 or anti-PD-L2 combination treatment addresses especially the T-cells to activate and repolarize them. Since brain metastases grow in the same immune-compartment as pirmary brain tumors, patients suffering from secondary brain tumors also benefit from the combination treatment.

In one embodiment, the combination medicament comprises an IL-12 polypeptide having a biological activity of IL-12 provided as a fusion protein comprising the amino acid of human p40, the amino acid sequence of human p35 and the crystallisable fragment of human IgG4, said IL-12 polypeptide being formulated as a dosage form for intratumoural delivery. According to this embodiment, the combination medicament further comprises an immunoglobulin G raised against CTLA-4 or PD-1 as a non-agonist CTLA-4 ligand and/or a non-agonist PD-1 ligand formulated as a dosage form for systemic delivery. According to this embodiment, the combination medicament is provided for the treatment of malignant neoplastic disease, particularly for glioma, glioblastoma multiforme, meningioma, melanoma, pancreatic cancer, lung cancer, prostate cancer or bladder cancer.

According to yet another aspect of the invention, an IL-12 polypeptide having a biological activity of IL-12, and a non-agonist CTLA-4 ligand and/or non-agonist PD-1 ligand are used in the manufacture of a combination medicament for use in a method of therapy of a malignant neoplastic disease, particularly of glioma and other solid tissue tumours, such as glioblastoma multiforme, meningioma, melanoma, pancreatic cancer, lung cancer, prostate cancer or bladder cancer.

According to yet another aspect of the invention, a method is provided for treating a patient suffering from malignant neoplastic disease, particularly glioma and other solid tissue tumours, comprising the administration of an IL-12 polypeptide having a biological activity of IL-12, and a non-agonist CTLA-4 ligand and/or a non-agonist PD-1 ligand to said patient.

According to an alternative aspect of the invention, a combination therapy comprises an IL-12 nucleic acid expression vector encoding an encoded IL-12 polypeptide having a biological activity of IL-12, and a T cell inhibition blocker agent selected from
- a non-agonist CTLA-4 ligand and
- a non-agonist PD-1 or PD-L1 or PD-L2 ligand.

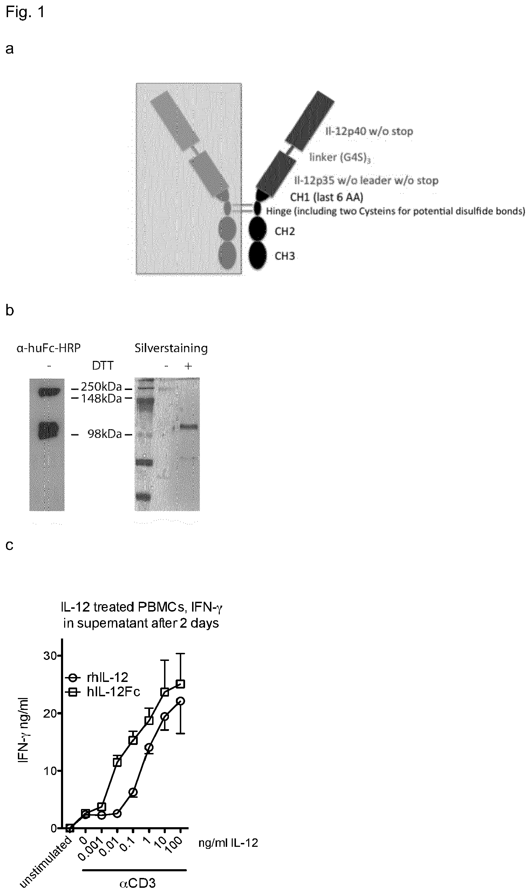

The CTLA-4 ligand and a non-agonist PD-1 ligand may be embodied by polypeptides, particularly by antibodies, as set forth above. One non-limiting example for an encoded IL-12 polypeptide is a crystallisable immunoglobulin G fragment fused to the IL-12 constituent polypeptide chains, human IL-12 or a functional equivalent thereof. One non-limiting example is a fusion construct having the constituent polypeptides of IL-12 linked by a short amino acid sequence as depicted in Fig. 1, the amino acid sequence for which is given as SEQ ID 01 and the encoding nucleic acid sequence is given as SEQ ID 07.

According to yet another aspect of the invention, a polypeptide peptide is provided comprising
a. a polypeptide sequence at least 95% identical to the sequence of human p35 (SEQ ID 05), and
b. a polypeptide sequence at least 95% identical to the sequence of human p40 (SEQ ID 06) and
c. a human immunoglobulin G subgroup 4 crystallisable fragment.

In some embodiments, the polypeptide comprises or essentially consists of a sequence at least 95%, 96%, 97%, 98%, 99% identical to SEQ ID 01, or is SEQ ID 01.

The advantage of using fusion proteins cytokines and the crystallisable fragment of immunoglobulins rather than the recombinant cytokine is improved pharmacokinetics (Belladonna et al. J Immunol 168, 5448-5454 (2002); Schmidt, Curr Opin Drug Discov Devel 12, 284-295 (2009); Eisenring et al., Nat Immunol 11, 1030-1038 (2010)).

The IL-12 nucleic acid expression vector according to this aspect of the invention may, by way of non-limiting example, be a "naked" DNA expression plasmid comprising a nucleic acid sequence encoding the IL-12 polypeptide under control of a promoter sequence operable in a human tumour cell, for delivery into the tumour, for example by intracranial injection. The IL-12 nucleic acid expression vector may similarly be a viral vector, for example an adeno-associated virus, an adenovirus, a lentivirus or a herpes virus.

Such IL-12 nucleic acid expression vector may be provided as a dosage form for intratumoural delivery in combination with a protein non-agonist CTLA-4 ligand and/or a non-agonist PD-1 ligand as set forth above. Similarly, the scope of the present invention encompasses the use of such IL-12 nucleic acid expression vector, in combination with a non-agonist CTLA-4 ligand and/or a non-agonist PD-1 ligand, in a method of making a combination medicament for use in therapy of malignant neoplastic disease, particularly glioma, glioblastoma multiforme, meningioma, melanoma, pancreatic cancer, lung cancer, prostate cancer or bladder cancer. Likewise, a method is provided for treating a patient suffering from malignant neoplastic disease, particularly glioma or other solid tissue tumours, comprising the administration of an IL-12 nucleic acid expression vector having a biological activity of IL-12, and a non-agonist CTLA-4 ligand and/or a non-agonist PD-1 ligand to said patient.

The invention further encompasses the following items:
1. A combination medicament, comprising
   - an IL-12 polypeptide, or a nucleic acid expression vector comprising a sequence encoding said IL-12 polypeptide, and
   - a T cell inhibition blocker agent selected from
      - a non-agonist CTLA-4 ligand and
      - a non-agonist PD-1 ligand
   for use in the therapy of a malignant neoplastic disease, wherein
   - said IL-12 polypeptide, or said nucleic acid expression vector, is provided by administration into a tumour, into the vicinity of a tumour, or to the lymph node associated with a tumour.
2. A combination medicament according to item 1, wherein said IL-12 polypeptide comprises
   a. a polypeptide sequence at least 95% identical to the sequence of human p35 (SEQ ID 05), and
   b. a polypeptide sequence at least 95% identical to the sequence of human p40 (SEQ ID 06).
3. A combination medicament according to item 2, wherein said IL-12 polypeptide comprises an immunoglobulin G crystallisable fragment.
4. A combination medicament according to item 2 or 3, wherein said IL-12 polypeptide comprises a human immunoglobulin G subgroup 4 crystallisable fragment.
5. A combination medicament according to item 4, wherein said IL-12 polypeptide comprises
   a. an immunoglobulin G crystallisable fragment and a recombinant or synthetic human IL-12 sequence, or
   b. a sequence at least 95% identical to SEQ ID 01.
6. A combination medicament according to any of the above items, wherein said T cell inhibition blocker agent is selected from
   - a non-agonist polypeptide CTLA-4 ligand and
   - a non-agonist polypeptide PD-1 ligand.
7. A combination medicament according to any of the above items, wherein
   - said non-agonist CTLA-4 ligand is a gamma immunoglobulin that binds to CTLA-4 and/or
   - said non-agonist PD-1 ligand is a gamma immunoglobulin that binds to PD-1.
8. A combination medicament according to any of the above items, wherein said IL-12 polypeptide is provided as a dosage form for intratumoural injection.
9. A combination medicament according to any of the above items, wherein said T cell inhibition blocker agent is provided as a dosage form for intravenous injection or local application.
10. A combination medicament according to at least one of items 1 to 9 for use in a method of therapy of glioma, glioblastoma multiforme, meningioma, secondary brain cancer, brain metastases, melanoma, pancreatic cancer, lung cancer, prostate cancer or bladder cancer.
11. A combination medicament according to item 1, wherein
   - said IL-12 polypeptide is a fusion protein comprising the amino acid of human p40, the amino acid sequence of human p35 and the crystallisable fragment of human IgG4,
   - said IL-12 polypeptide is provided as a dosage form for intratumoural delivery, and wherein
   - said T cell inhibition blocker agent is an immunoglobulin G provided as a dosage form for systemic delivery,
   for the treatment of malignant neoplastic disease. particularly glioma, glioblastoma multiforme, meningioma, secondary brain cancer, brain metastases, melanoma, pancreatic cancer, lung cancer, prostate cancer or bladder cancer.
12. The combination medicament according to item 11, wherein T cell inhibition blocker agent is selected from
   - a non-agonist CTLA-4 antibody and
   - a non-agonist PD-1 antibody.
13. A combination medicament according to at least one of items 1 to 9, wherein said nucleic acid expression vector is an adenovirus, an adeno-associated virus, a lentivirus or a herpesvirus.
14. Use of an IL-12 polypeptide having a biological activity of IL-12, and a T cell inhibition blocker agent selected from a non-agonist CTLA-4 ligand and a non-agonist PD-1 ligand, in the manufacture of a combination medicament for use in the therapy of a malignant neoplastic disease, glioma, glioblastoma multiforme, meningioma, secondary brain cancer, brain metastases, melanoma, pancreatic cancer, lung cancer, prostate cancer or bladder cancer, wherein the IL-12 polypeptide is provided as a dosage form for administration into a tumour, into the vicinity of a tumour, or to the lymph node associated with a tumour.
15. A method of treating a patient suffering from malignant neoplastic disease, comprising the administration into a tumour, into the vicinity of a tumour, or to the lymph node associated with a tumour, of an IL-12 polypeptide having a biological activity of IL-12, and the administration of a T cell inhibition blocker agent selected from a non-agonist CTLA-4 ligand and a non-agonist PD-1 ligand.
16. A polypeptide comprising
   a. a polypeptide sequence at least 95% identical to the sequence of human p35 (SEQ ID 05), and
   b. a polypeptide sequence at least 95% identical to the sequence of human p40 (SEQ ID 06) and
   c. a human immunoglobulin G subgroup 4 crystallisable fragment.
17. The polypeptide of item 16, having a sequence at least 95% identical to SEQ ID 01.
18. The polypeptide of items 16 or 17 for use as a medicament.
19. The polypeptide of items 16 or 17 for use in cancer therapy.
20. The polypeptide of items 16 or 17 for use in therapy of glioma, glioblastoma multiforme, meningioma, secondary brain cancer, brain metastases, melanoma, pancreatic cancer, lung cancer, prostate cancer or bladder cancer.

### Brief description of the Figures

- Fig. 1a: shows the structure and sequence of the fusion protein given in SEQ ID 01.The subunits p40 and p35 of IL-12 are depicted as rectangles. These subunits are connected by a linker (G₄S)₃. The subunits CH2, CH3 and the last six amino acids of CH1 of the crystallizable fragment of the immunoglobulin are shown as oblong circles.
- Fig. 1b: shows the fusion protein given SEQ ID 01, left picture shows an immunoblot using reducing conditions, developed with an HRP-coupled polyclonal anti-human Fc antibody, right picture shows a silver staining of the fusion protein under non-reducing (DTT -) and reducing conditions (DTT +)
- Fig. 1c: shows IFN-γ production in human peripheral blood monocytic cells (PBMCs) as assessed by enzyme linked immunosorbent assay (ELISA). Cells were stimulated either with commercially available heterodimeric recombinant human IL-12 (rhIL-12) or with the purified fusion protein given SEQ ID 01 (hIL-12Fc) in the presence of an antibody directed against CD3 (polyclonal T-cell stimulation). Unstimulated: neither IL-12 stimulation nor anti-CD3 stimulation (baseline control). Experiment was performed in triplicates, error bars denote s.e.m. data representative of three independent experiments

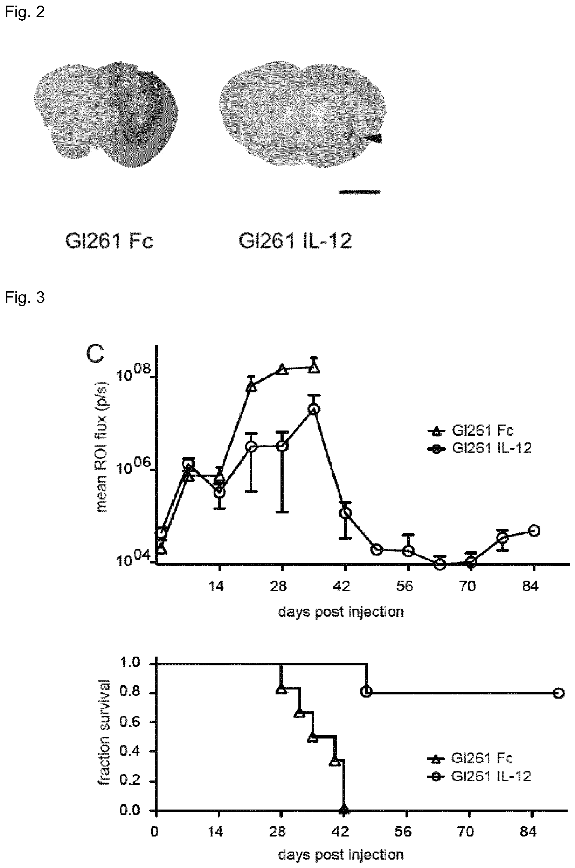

- Fig. 2: shows immunohistochemistry of formalin fixed tumour sections obtained from syngeneic C57/BI6 mice 5 weeks after challenge with 2x10⁴ GI261 IL-12Fc or GI261 Fc cells and stained with antibody against F4/80, counterstain hematoxylin (representative examples, n=6 mice per group). Scale bar indicates 2 mm, arrowhead indicates residual GI261 IL-12Fc (SEQ ID 02) tumour.
- Fig. 3: shows non-invasive Bioluminescence imaging (BLI) of mouse glioma in syngeneic C57/BI6 mice (n = 5-6 mice per group) after implantation of 2x10⁴ GI261 cells constitutively expressing *photinuspyralis* luciferase and releasing a fusion protein of IL-12 and the crystallizable fragment of mouse immunoglobulin G3 (GI261 IL-12Fc, (SEQ ID 02)) or Fc alone as control (GI261 Fc). Upper panel: Quantification of tumour growth which correlates to photon flux (p/s) in the region of interest (ROI) versus the days post injection of the modified glioma cells. Lower panel: Kaplan-Meier survival analysis. Data are representative of 2 independent experiments.

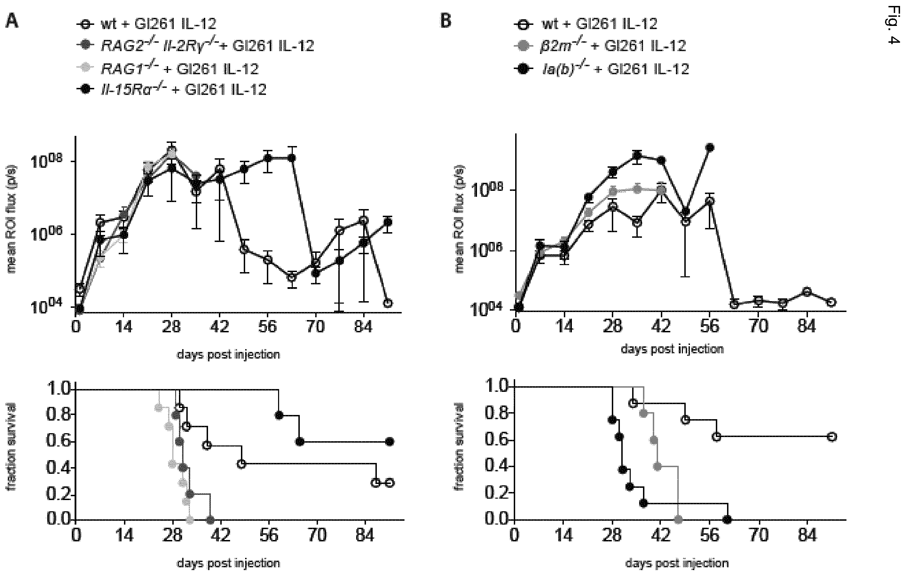

- Fig. 4: shows non-invasive Bioluminescence (BLI) imaging of mouse glioma in WT animals and different mouse mutants (n = 5-7mice per group) after implantation of 2x10⁴ GI261 cells constitutively expressing *photinuspyralis* luciferase and releasing a fusion protein of IL-12 and the crystallizable fragment of mouse immunoglobulin G3 (GI261 IL-12Fc, (SEQ ID 02)). Upper panel: Quantification of tumour growth via BLI imaging versus the days post injection of the modified glioma cells. Lower panel: Kaplan-Meier survival analysis. **A)** GI261 IL-12Fc were implanted in mice lacking T and B cells (R*ag1*^{*-*/*-*}) or NK cells (*II-15ra*^{*-*/*-*}) or lacking both T-, B-, NK cells and lymphoid tissue inducer like cells (*Rag2*^{*-*/}*⁻ II2rg^{-l-}*). **B)** GI261 IL-12Fc were implanted in mice deficient for MHCII (*Ia(b)*^{*-*/*-*}) and MHCI (*β2m*^{*-*/*-*}).(n=5-8 mice/group), lacking CD4 or CD8 positive T-cells, respectively. Data are representative of 2 independent experiments.

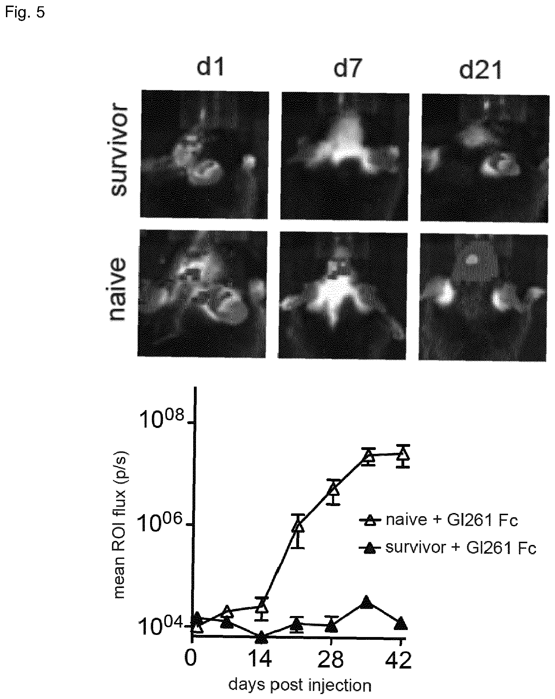

- Fig. 5: shows T cell memory formation in surviving wt animals that had been previously challenged with 2x10⁴ GI261 IL-12Fc (SEQ ID 02) cells. Examples for bioluminescence emitted from the brains of surviving wt animals that had been rechallenged with GI261 Fc cells compared to naive wt animals is shown (upper panel, days 1,7 and 21 post rechallenge shown). Furthermore, bioluminescence of the tumours is shown in photons per second (p/s) in the region of interest (ROI) versus the days post injection of the modified glioma cells (lower panel). A rapid rejection of the control tumours in surviving wt animals was observed. While the measured luminescence at day 1 suggested identical seeding across the two groups, only the naive mice exhibited a measurable signal at day 7 onwards, suggesting a rapid and effectively clearing anti-glioma memory response now independent of ectopically expressed pro-inflammatory cytokines (namely IL-12Fc, (SEQ ID 02)).(n=4-6 mice/group). Data are representative of 2 independent experiments.

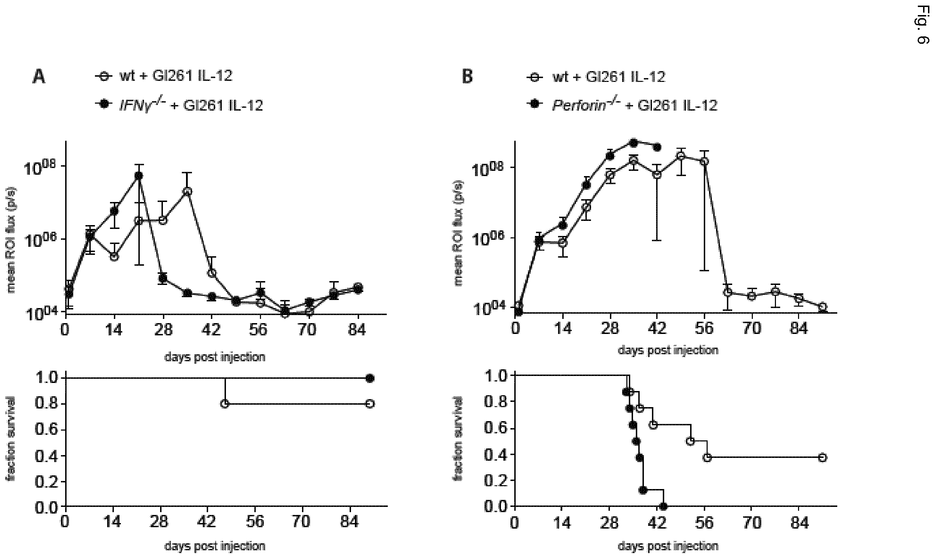

- Fig. 6: shows non-invasive Bioluminescence (BLI) imaging of mouse glioma in different mouse mutants (n = 4-8 mice per group) after implantation of 2x10⁴ GI261 Fccells constitutively expressing *photinuspyralis* luciferase and releasing a fusion protein of IL-12 and the crystallizable fragment of mouse immunoglobulin G3 (GI261 IL-12Fc (SEQ ID 02)). A) wt (open circles) and *IFNγ*^{*-*/*-*} (black circles) animals B) wt (open circles) and *Perforin*^{*-*/*-*} (black circles) animals. Quantification of tumour growth which correlates to photon flux (p/s) in the region of interest (ROI) versus the days post injection of the modified glioma cells are shown (upper panel). Lower panel: Kaplan-Meier survival analysis. Data are representative of 2 independent experiments.

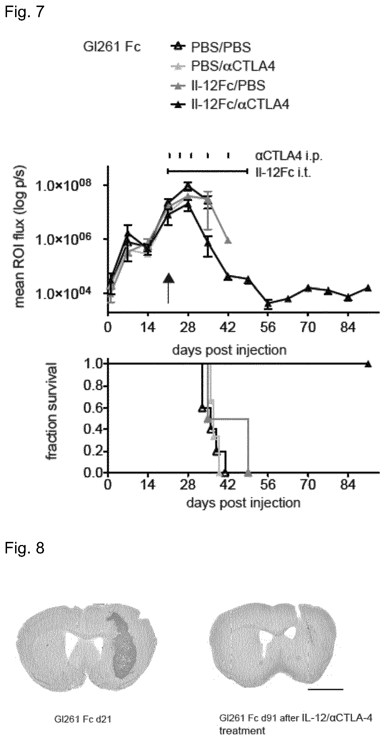

- Fig. 7: shows tumour growth in wt mice inoculated with 2x10⁴ GI261 Fc cells. Treatment started at day 21 (arrows). Osmotic minipumps delivering IL-12Fc (SEQ ID 02) (or PBS) into the tumour were implanted into glioma bearing animals. Animals received i.p. injections of αCTLA-4 blocking antibodies or PBS starting at day 22, followed by injections as indicated in figure. Upper graph: quantification of ROI photon flux of tumour bearing wt animals receiving the indicated treatment. Lower graph: Kaplan-Meier survival analysis of the animals above; PBS/PBS vs IL-12Fc/αCTLA-4 p=0.0045, PBS/PBS vs IL-12Fc/PBS p=0.3435, PBS/ αCTLA4 vs IL-12Fc/αCTLA-4 p=0.0101; Log-rank (Mantel-Cox) Test. Data representative of three independent experiments with 2-5 animals per group
- Fig. 8: shows immunohistochemistry of tumour sections obtained from syngenic C57/BI6 mice after challenge with GI261 Fc cells at day 21 and after local administration of IL-12Fc (SEQ ID 02) in combination with systemic CTLA-4 blockade as described in Example 5. The sections were stained with Hematoxylin and Eosin. Scale bar indicates 2 mm.

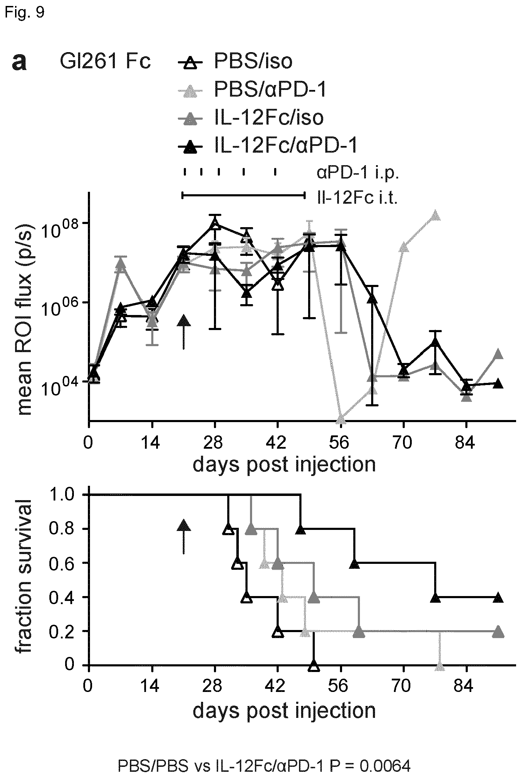

- Fig. 9: shows tumour growth in wt mice inoculated with 2x10⁴ GI261 Fc cells. Treatment started at day 21 (arrows). Osmotic minipumps delivering IL-12Fc (SEQ ID 02) into the tumour were implanted into glioma bearing animals. Animals received i.p. injections of αPD-1 blocking antibodies or isotype control antibodies starting at day 22, followed by injections as indicated in figure. Upper graph: quantification of ROI photon flux of tumour bearing wt animals receiving the indicated treatment. Lower graph: Kaplan-Meier survival analysis of the animals above; PBS/isotype vs IL-12Fc/αPD-1 p=0.0064, Log-rank (Mantel-Cox) Test. Data representative of one experiment with 5-6 animals per group.

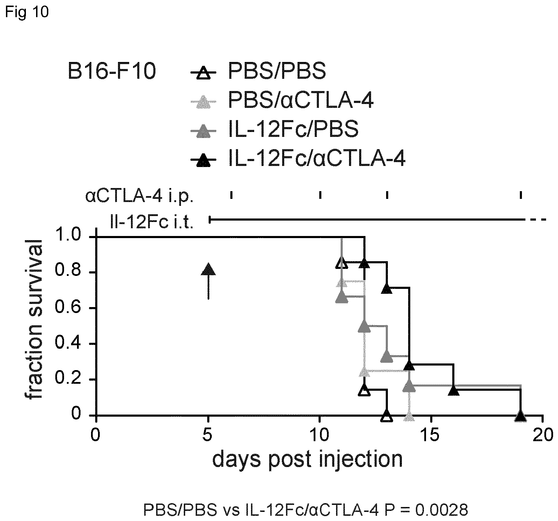

- Fig. 10: shows tumour growth in wt mice in inoculated with 50 B16-F10 cells. Treatment started at day 5 (arrow). Osmotic minipumps delivering IL-12Fc (SEQ ID 02) into the tumour were implanted into glioma bearing animals. Animals received i.p. injections of αCTLA-4 blocking antibodies or PBS starting at day 6, followed by injections as indicated in figure. Kaplan-Meier survival analysis PBS/PBS vs IL-12Fc/αCTLA-4 p=0.0028, Log-rank (Mantel-Cox) Test. Data representative of one experiment with 6 animals per group.
- Fig. 11: shows systemic administration of recombinant heterodimeric IL-12 in combination with CTLA4 blockade 2x10⁴ GI261 Fc cells were injected into the right striatum of wt mice and tumor growth was followed for 90 days. Systemic treatment: at day 21 (arrow), tumor bearing animals were treated initially with 200µg αCTLA-4 mouse IgG2b (9D9) (filled light grey triangles, n=8), 200ng of recombinant heterodimeric IL-12 (rIL-12) (filled dark grey triangles, n=9) or a combination of both (filled black triangles, n=9) injected intraperitoneal (i.p.). The control group received phospate buffered saline (PBS) (filled open triangles, n=7). Treatment was sustained with 100µg αCTLA-4 or 100ng rIL-12 or a combination of both 3 times / week until the end of the experiment. Upper graph: quantification of ROI photon flux of tumor bearing wt animals receiving the indicated treatment. Lower graph: Kaplan-Meier survival analysis of the animals above; Log-rank (Mantel-Cox) Test was used to calculate the p-values indicated; Pooled data from two independent experiments.

### Examples

### Methods

### Animals

C57BL/6 mice were obtained from Janvier; *b2m*^{*-*/}*⁻, lla(b)*^{*-*/}*⁻, ll12rb2^{-l-}, Rag1*^{*-*/}*⁻, Rag2*^{*-*/}*⁻ll2rg^{-l-}, Prf1*^{*-*/*-*} and *lfng*^{*-*/*-*} mice were obtained from Jackson Laboratories. *ll15ra*^{*-*/*-*} mice were provided by S. Bulfone-Paus. All animals were kept in house under specific pathogen-free conditions at a 12hour light/dark cycle with food and water provided *ad libitum.* All animal experiments were approved by the Swiss Cantonary veterinary office (16/2009).

### Mouse tumour cell lines

C57/BI6 murine glioma (GI261) cells (kindly provided by A. Fontana, Experimental Immunology, University of Zürich) were transfected with pGI3-ctrl (Promega) and pGK-Puro (kindly provided by T. Buch, Technical University Munich). Linearized constructs were electroporated in a 10:1 ratio using an eppendorf multiporator, then selected with 0.8µg/ml puromycin (Sigma-Aldrich) to generate luciferase-stable GI261 cells. A single clone was isolated by limiting dilution and passaged in vivo by intracranial tumour inoculation, followed by tumour dissociation after 4 weeks and re-selection in 0.8µg/ml puromycin. Subsequently, cells were electroporated with pCEP4-mIgG3, pCEP4-mll-12mIgG3 (SEQ ID 09) and pCEP4-mll-23mIgG3 (SEQ ID 08) (Eisenring et al, 2010) and bulk-selected with 0.8µg/mlpuromycin and 0.23mg/ml hygromycin (Sigma-Aldrich). Cytokine production was detetected by ELISA (OptEIA Il-12/23p40, BD Pharmingen) and rt-PCR (IgG3fw: ACACACAGCCTGGACGC (SEQ ID 03) IgG3rev: CATTTGAACTCCTTGCCCCT (SEQ ID 04)). GI261 cells and derived cell lines were maintained in Dulbecco's modified Eagle's medium (Gibco, Invitrogen) supplemented with 10% fetal calf serum (FCS) in presence of selection antibiotics as indicated above at 37°C and 10% CO₂. B16-F10 murine melanoma cells were purchased from ATCC.

### Expression and purification of IL-12Fc

IL-12Fc (SEQ ID 02) was expressed in 293T cells after calcium phosphate-mediated transfection according to standard protocols with 45µg of vector DNA (pCEP4-mIL-12IgG3, SEQ ID 09)/15cm tissue culture plate. Supernatant was harvested 3 days and 6 days after transfection, sterile filtered and diluted 1:1 in PBS. The protein was purified using a purifier (AktaPrime) over a protein G column (1ml, HiTrap, GE Healthcare) eluted with 0.1 M glycine pH 2 and dialyzed over night in PBS pH 7.4. Concentration and purity of IL-12Fc (SEQ ID 02) was measured by ELISA (OptEIA Il-12/23p40, BD Pharmingen) and SDS-PAGE followed by silverstaining and immunoblotting. IL-12Fc was detected with a rat anti mouse IL-12p40 antibody (C17.8, BioExpress) and a goat anti-rat HRP coupled antibody (Jackson). The same procedure was used for the expression of human IL-12Fc (SEQ ID 01, 07).

### Characterization of human IL-12Fc

Concentration and purity of human IL-12Fc (SEQ ID 01) was measured by ELISA (Human IL-12 (p70), Mabtech, #2455-1H-6) and SDS-PAGE followed by silver staining and immunoblotting. The human IgG4 tag was detected with an HRP-coupled goat anti human IgG antibody (#A0170, Sigma). For functional characterization of human IL-12Fc (SEQ ID 01) PBMCs, acquired according to the ethical guidelines of the University of Zurich, were plated at 100'000 cells per well in RPMI medium supplemented with 10% fetal calf serum (FCS) in 96 well plates and stimulated with either recombinant human IL-12 (Peprotech) or human IL-12Fc (SEQ ID 01). Both cytokines were normalized to each other according to concentrations derived from human IL-12p70 ELISA (Mabtech, #2455-1H-6). PBMCs were stimulated in the presence of 1µg/ml of a mouse IgG2a anti-human CD3 antibody (OKT3, Bio-X-cell). After two days of culture in 5% CO₂ and 37°C, supernatant was harvested and subjected to an anti-human IFN-γ ELISA (Mabtech, #3420-1H-6).

### Orthotopic glioma inoculation

Briefly, 6-10 week old mice were i.p. injected with Fluniximin (Biokema, 5mg/kg body weight) before being anaesthesized with 3-5% isoflurane (Minrad) in an induction chamber. Their heads were shaved with an electric hair-trimmer. After being mounted onto a stereotactic frame (David Kopf Instruments), the animals' scalp was disinfected with 10% iodine solution and a skin incision was made along the midline. Anaesthesia on the stereotactic frame was maintained at 3% isoflurane delivered through a nose adaptor (David Kopf Instruments). Subsequently, a blunt ended syringe (Hamilton, 75N, 26s/2"/2, 5µl) was mounted on a microinjection pump on the manipulator arm and placed 1.5mm lateral and 1mm frontal of bregma. The needle was lowered into the manually drilled burr hole at a depth of 4mm below the dura surface and retracted 1mm to form a small reservoir. Using the microinjection pump (UMP-3, World Precision Instruments Inc.) 2x10⁴ cells were injected in a volume of 2µl at 1µl/min. After leaving the needle in place for 2min, it was retracted at 1mm/min. The burr hole was closed with bone wax (Aesculap, Braun) and the scalp wound was sealed with tissue glue (Indermil, Henkel).

### In vivo bioluminescent imaging

Tumour bearing mice were carefully weighed, anaesthesized with isoflurane (2-3%) and injected with D-Luciferin (150mg/kg body weight, CaliperLifesciences). Animals were transferred to the dark chamber of a Xenogen IVIS 100 (CaliperLifesciences) imaging system, anaesthesia was maintained at 2% isoflurane via nosecones. 10min after injection luminescence was recorded. Data was subsequently analyzed using Living Image 2.5 software (CaliperLifesciences). A circular region of interest (ROI; 1.46cm Ø) was defined around the animals' head and photon flux of this region was read out and plotted.

### Treatment of established gliomas

At d21 after implantation of the glioma cells, the tumour bearing animals were evenly distributed among experimental groups based on their ROI-photon flux. Animals with an ROI flux of less than 1x10⁵ p/s were considered as non-takers and excluded. 40-48h prior to implantation (2 days before beginning of treatment), osmotic pumps (Model 2004, 0.25µl/h; Alzet) were filled with murine IL-12Fc (SEQ ID 02, 8.33 ng/µl in PBS) or PBS alone and primed at 37°C in PBS. Immediately prior to surgery, mice were injected with Fluniximini.p. (Biokema, 5mg/kg body weight). Mice were anaesthesized with 3-5% isoflurane, the scalp was disinfected and a midline incision was made. The previous burr hole of the glioma injection was located, the bone wax and periost removed and the pump placed into a skin pouch formed at the animal's back. The infusion cannula was lowered through the burr hole 3mm into the putative center of the tumour. The cannula was connected to the pump (brain infusion kit III 1-3mm, Alzet) via a silicon tube and held in place with cyanoacrylate adhesive. The skin was sutured with a 4-0 nylon thread. Following surgery, mice were treated for 3 days with 0.1% (v/v) Borgal (Intervet) in the drinking water. Pumps were explanted at day 49. Five doses of anti mouse-CTLA-4 mouse-IgG2b antibodies (clone 9D9, bio-X-cell; Peggs et al.;J Exp Med 206, 1717-1725 (2009)) or an equivalent volume of PBS were i.p. injected at days 22 (200µg), 26 (100µg), 29 (100µg), 35 (100µg) and 42 (100µg).

Alternatively, animals received anti-mouse-PD-1 rat IgG2a (clone RMP1-14, bio-X-cell) or rat IgG2a isotype control antibodies (clone 2A3, bio-X-cell) for the experiment depicted in figure 9. Dosing schedule and application route was identical with the experiment depicted in figure 7. For treatment of established B16-F10 derived brain tumours, pumps were implanted at day 5 post injection, anti mouse-CTLA-4 mouse-IgG2b antibodies (clone 9D9, bio-X-cell; Peggs et al.;J Exp Med 206, 1717-1725 (2009)) or an equivalent volume of PBS were i.p. injected at days 6 (200µg), 11 (100µg), 13 (100µg) and 19 (100µg).

### Survival analysis of tumour bearing animals

Tumour bearing animals were monitored by BLI, checked for neurological symptoms and weighed weekly until day 21 post glioma inoculation. GI261 Fc animals exhibiting an ROI flux of less than 1x10⁵ p/s at day 21 were considered as non or slow-tumour takers and excluded from the survival analysis (5-10%). From day 21 onwards animals were checked daily. Animals that showed symptoms as apathy, severe hunchback posture and/or weight loss of over 20% of peak weight were euthanized. B16-F10 tumour bearing mice were scored daily starting at day 5 until the end of experiment according to the same scheme.

### Histology

For histology, animals were euthanized with CO₂, transcardially perfused with ice-cold PBS and decapitated. Whole brains were carefully isolated, fixed in 4% Formalin, embedded in Paraffin and 3µm sections were processed for HE staining and/or immunohistochemistry to detect F4/80 (BM8; BMA biomedicals). Primary antibodies were detected with HorseRadish Peroxidase- coupled secondary antibodies. Staining was visualized with 3,3'-Diaminobenzidin (DAB) as the HRP substrate. Pictures were generated using an Olympus BX41 light microscope equipped with an Olympus ColorViewlllu camera and Olympus cell^B image acquisition software. Overviews of whole brains slices were cropped using Adobe Photoshop CS3.

### Statistical analysis

For statistical analysis of Kaplan-Meier survival curves, a Log-rank (Mantel-Cox) Test was used to calculate the p-values indicated in respective figures. P values of less than 0.05 were considered statistically significant. Analysis was performed with GraphPad Prism version 5.0a for Mac OSX (GraphPad Software Inc).

### Example 1: Intratumoural expression of IL-12Fc promotes clearance of experimental gliomas

We have designed and cloned a fusion protein consisting of the p40 subunit of human IL-12 linked via a flexible peptide linker to the p35 subunit. This single chain construct was then fused to the constant region of human IgG4 heavy chain (Fig. 1A). We termed this human single chain fusion protein IL-12Fc (SEQ ID 01.) We expressed this protein in HEK293 human embryonic kidney cells and detected a dimeric as well as a monomeric form under native conditions. Under reducing conditions only the monomeric form is detectable (Fig. 1B). IL-12Fc (SEQ ID 01) has similar functional properties as commercially available heterodimeric IL-12 (purchased from Peprotech). To determine if IL-12Fc (SEQ ID 01) could be suitable to overcome the local immunosuppressive environment induced by gliomas and to shed light on the effector mechanisms involved, we expressed a murine version (Belladonna et al. J Immunol 168, 5448-5454 (2002), IL-12Fc (SEQ ID 02)) of this cytokine in GI261 mouse glioma cells. To measure intracranial tumour growth non-invasively via bioluminescence imaging (BLI) we first generated a GI261 line that constitutively expresses *photinus pyralis* luciferase. We termed this cell line GI261-luc. We next modified this cell line to continuously release a fusion protein of IL-12 and the crystallizable fragment of mouse immunoglobulin G3 (IL-12Fc; SEQ ID 02, 09) or Fc (SEQ ID 08) alone as a control (termed 'GI261 IL-12Fc'and 'GI261 Fc', respectively). The chosen murine protein sequence of the fusion construct is homologous to the human variant (SEQ ID 01) and consists of the subunits p40 and p35 of IL-12 which are connected by a linker (G4S)₃ and the subunits CH2, CH3 and the last six amino acids of CH1 of the crystallizable fragment of IgG3, whereas CH1 and CH2 are connected by a hinge region. Vectors for expression of the control fragment and the IL-12 fusion construct are depicted in SEQ ID 08 and 09, respectively. The intention of using fusion proteins rather than the recombinant cytokine was to see whether they would exhibit improved pharmacokinetics. We confirmed secretion of IL-12Fc (SEQ ID 02) by ELISA for the subunits p40 and p70. We further confirmed expression of the Fc tail by RT-PCR. When implanted intracranially into the right striatum, luminescence readings and tumour volume as assessed by stereologic methods showed a robust correlation (data not shown).

We next implanted GI261 IL-12Fc and Fc into the right striatum of syngenic C57BI/6 mice and followed tumour growth via non invasive bioluminescence imaging (BLI). After an initial increase in luminescence all groups showed a depression around day 14 post injection. Animals bearing Fc-expressing tumours exhibited a steep increase in BLI and soon reached withdrawal criteria, sometimes even before day 35 post injection. In contrast, BLI-readings for animals that had been injected with IL-12Fc expressing GI261 tumours dropped to levels close to the detection limit at day 21 onwards (data not shown). In agreement with this observation, we could only detect a residual tumour in some animals in this group, while Fc control-injected animals showed robust tumour formation when analyzed histologically (Fig. 2). When we followed animals that had been implanted with GI261 IL-12Fc or GI261 Fc cells for up to 90 days, we observed rejection of the tumour in a high proportion of mice bearing IL-12Fc secreting tumours after an initial establishment (
Fig. 3).

### Example 2: T-cells are the major effector cell type of IL-12Fc mediated glioma rejection.

To confirm that the secretion of IL-12Fc by GI261 IL-12Fc acts on the host rather than the tumour cells themselves, we observed the growth of GI261 IL-12Fc and GI261 Fc to be the same in mice lacking the receptor to IL-12. The unbridled growth of GI261 IL-12 in *IL-12rβ2*^{*-*/*-*} animals demonstrates that IL-12Fc acts specifically on a cell type in the recipient mouse (data not shown). T and NK cells are among the most prominent IL-12 responsive leukocytes. To systematically test the functional relevance of the IL-12Fc mediated influx of these cells, we challenged a series of mouse mutants with intracranial GI261 IL-12Fc. We implanted GI261 IL-12Fc cells in mice that lack T and B cells (*Rag1*^{*-*/*-*}) or conventional Nk-cells (*II-15ra^{-l-}*) or in mice lacking both T-, B-, Nk-cells and lymphoid tissue inducer-like cells *(Rag2*^{*-*/}*⁻ ll2rg*^{*-*/*-*}) (Fig. 4A). After an initial lag phase until day 14 after injection, all groups exhibited a strong increase in luminescence until day 28, reflecting strong tumour growth. Between days 28 and 42 most of the animals succumbed to the tumours. Only wt and *II-15ra*^{*-*/*-*} mice were able to control the tumour and show a significantly prolonged survival compared to *Rag2*^{*-*/}*⁻ ll2rg^{-l-}* and *Rag1*^{*-*/*-*} animals. While T or B-cells appeared to be crucial for IL-12Fc mediated glioma rejection, the ability of *II-15ra*^{*-*/*-*} mice to reject GI261 IL-12 indicates that NK cells were largely expendable.

We next investigated the contribution of CD4- and CD8 positive T-cells using MHCII (*Ia(b)*^{*-*/*-*}) and MHCI (*β2m*^{*-*/*-*}) deficient mice. In contrast to wt mice, *Ia(b)*^{*-*/*-*} mice lacking CD4 T cells could not control GI261 IL-12Fc tumours, and *β2m*^{*-*/*-*} mice succumbed to the glioma shortly afterwards (Fig.4B).The survival in both mutant groups was shortened compared to the wildtype group. These data clearly demonstrate that IL-12Fc mediated tumour rejection is dependent on the activity of T cells including helper T cells and CTLs.

### Example 3: The antitumoural memory response is independent of ectopically expressed IL-12Fc.

To further investigate the character of the T-cell dependent tumour control, we tested the surviving wt animals that had been previously challenged with GI261 IL-12Fc cells for T cell memory formation (Fig. 5). The animals were treated as described in Fig. 3 / example 1. In contrast to the primary challenge, we now injected GI261 Fc cells into the contralateral hemisphere of survivors or naive wt animals. We observed a rapid rejection of the control tumours within days. While the measured luminescence at day 1 suggested identical seeding across the two groups, only the naive mice exhibited a measurable signal at day 7 onwards, suggesting a rapid and effectively clearing anti-glioma memory response now independent of ectopically expressed pro-inflammatory cytokines.

### Example 4: CTLs are the main effector cells of IL-12Fc-mediated glioma rejection.

It is well established that IL-12 polarizes naive T-cells to adopt a T_{H}1 phenotype (Trinchieri, Nat Rev Immunol 3, 133-146 (2003)). To shed further light on the mechanistic underpinnings underlying the IL-12 induced rejection of experimental glioma, we challenged mice deficient in the T_{H}1 hallmark cytokine IFN-γ (*lfng*^{*-*/*-*}) with IL-12Fc expressing GI261 cells (Fig. 6A). The animals were treated as described in Fig. 3/ Example 1. To our surprise we observed a similar tumour rejection as in wt animals, suggesting that the mechanism of rejection is independent of IFN-γ. Conversely, IL-12 also stimulates the cytotoxic activity of CTLs. When we analyzed the role of Perforin, a cytolytic molecule primarily expressed on CD8⁺ CTLs and Nk-cells, we observed a clear difference in survival curves. (Fig. 6B). Perforin is a cytolytic molecule primarily expressed by CD8⁺ CTLs and NK cells but also CD4⁺ T-cells. To further investigate the mechanism of IL-12Fc induced rejection of glioma, perforin-deficient mice (*prf1*^{*-*/*-*}) were challenged with IL-12Fc expressing GI261 cells. In contrast to *lfng*^{*-*/*-*}, Perforin deficient animals (*prf1*^{*-*/*-*}) were not able to control the tumour. This further supports the notion that CTLs are the main effector cells of IL-12Fc mediated glioma rejection. A clear difference in the survival curves of wt and *prf1*^{*-*/*-*} was observed.

### Example 5: Local administration of IL-12Fc in combination with systemic CTLA-4 blockade is effective against advanced stage experimental gliomas

To further boost and prolong the activated phenotype of T-cells, we blocked the co-inhibitory molecule CTLA-4 via neutralizing antibodies in the next set of experiments. IL-12 was administered locally to mice with advanced stage tumours. Treatment was administered to animals that had been challenged with GI261 Fc 21 days before and that already exhibited strong bioluminescence signals, indicating an advanced stage of glioma growth. Local treatment: At day 21, osmotic minipumps delivering 50 ng IL-12Fc/day (or PBS) into the tumour were implanted into glioma bearing animals. After 28 days (day 49 after tumour injection) the empty pumps were explanted from surviving animals. Systemic treatment: At day 22, tumour bearing animals received 200 µg αCTLA-4 mouse IgG2b (9D9) or PBS i.p. Treatment was sustained with 100 µg αCTLA-4 at days 26, 29,35 and 42 (Fig. 7). Neither IL-12Fc, nor anti-CTLA-4 alone conferred any significant survival advantage. Strikingly, the combination of local IL-12Fc administration directly into the tumour site in combination with systemic CTLA-4 blockade led to a full remission of the tumour (Fig. 8). 90 days after inoculation, histologic assessment of the brain tissue of surviving animals did not show any signs of demyelination or infiltrates. Local IL-12Fc administration in combination with systemic PD-1 blockade also led to a significant increase in surviving animals, the frequency was however lower than with systemic CTLA-4 blockade (Fig. 9). The above described combination therapy (Fig. 7) confers a significant survival advantage even in the case of intracranial growth of B16-F10 syngeneic murine melanoma cells (Fig. 10). This is not a perfect model for secondary brain tumours since it is skipping various steps of metastasis formation. Even in this more aggressive situation the combination treatment prolongs survival.

Preventive treatment of tumours in preclinical models may allow the study of immunological mechanisms and the interactions between tumour cells and tumour microenvironment. However, preventive therapy is of limited clinical relevance in the translation to treat cancer patients. We thus decided to choose an exceptionally late timepoint for intervention in a progressing and aggressive disease model. To closely mimic a clinical situation, we allowed the tumour to progress to a size that is highly likely to cause significant neurological symptoms in humans. Here, monotherapy with locally applied (intratumoural) IL-12 had a minimal albeit significant survival effect. We already observed a weak synergistic effect when we combined systemic IL-12 treatment with systemic CTLA-4 blockade. When local IL-12 infusion was combined with systemic CTLA-4 blockade, the anti-glioma effect was striking.

## Claims

1. An IL-12 nucleic acid expression vector encoding an IL-12 polypeptide for use in the treatment of a malignant neoplastic disease,
wherein the IL-12 nucleic acid expression vector is provided by administration into a tumour, into the vicinity of a tumour, or to the lymph node associated with a tumour,
and wherein the IL-12 nucleic acid expression vector is administered in combination with a non-agonist ligand selected from the group consisting of an antibody, an antibody fragment, and an antibody-like molecule, wherein said non-agonist ligand is capable of binding to one of CTLA-4, PD-1, PD-L1 or PD-L2 with a dissociation constant of at least 10⁻⁷ M⁻¹, and wherein said non-agonist ligand is provided as a dosage form for systemic delivery.

2. The IL-12 nucleic acid expression vector for use according to claim 1, wherein said IL-12 polypeptide comprises
a. a polypeptide sequence at least 95% identical to the sequence of human p35 (SEQ ID 05), and
b. a polypeptide sequence at least 95% identical to the sequence of human p40 (SEQ ID 06).

3. The IL-12 nucleic acid expression vector for use according to claim 1 or 2, wherein said non-agonist ligand is a gamma immunoglobulin that binds to one of CTLA-4, PD-1, PD-L1 or PD-L2.

4. The IL-12 nucleic acid expression vector for use according to any one of claims 1 to 3, wherein said nucleic acid expression vector is an adenovirus, an Adeno-associated virus, a lentivirus or a herpesvirus.

5. The IL-12 nucleic acid expression vector for use according to any one of claims 1 to 4, wherein said malignant neoplastic disease is glioma, glioblastoma multiforme, meningioma, secondary brain cancer, brain metastases, melanoma, pancreatic cancer, lung cancer, prostate cancer or bladder cancer.

6. An IL-12 polypeptide for use in the treatment of a malignant neoplastic disease,
wherein the IL-12 polypeptide is provided by administration into a tumour, into the vicinity of a tumour, or to the lymph node associated with a tumour,
and wherein the IL-12 polypeptide is administered in combination with a non-agonist ligand selected from the group consisting of an antibody, an antibody fragment, and an antibody-like molecule, wherein said non-agonist ligand is capable of binding to one of CTLA-4, PD-1, PD-L1 or PD-L2 with a dissociation constant of at least 10⁻⁷ M⁻¹, and wherein said non-agonist ligand is provided as a dosage form for systemic delivery.

7. The IL-12 polypeptide for use according to claim 6, wherein said IL-12 polypeptide comprises
a. a polypeptide sequence at least 95% identical to the sequence of human p35 (SEQ ID 05), and
b. a polypeptide sequence at least 95% identical to the sequence of human p40 (SEQ ID 06).

8. The IL-12 polypeptide for use according to claim 6 or 7, wherein said non-agonist ligand is a gamma immunoglobulin that binds to one of CTLA-4, PD-1, PD-L1 or PD-L2.

9. The IL-12 polypeptide for use according to any one of claims 6 to 8, wherein said nucleic acid expression vector is an adenovirus, an Adeno-associated virus, a lentivirus or a herpesvirus.

10. The IL-12 nucleic acid expression vector for use according to any one of claims 6 to 9, wherein said malignant neoplastic disease is glioma, glioblastoma multiforme, meningioma, secondary brain cancer, brain metastases, melanoma, pancreatic cancer, lung cancer, prostate cancer or bladder cancer.

11. A gamma immunoglobulin that binds to one of CTLA-4, PD-1, PD-L1 or PD-L2, for use in the treatment of a malignant neoplastic disease,
wherein said gamma immunoglobulin is provided as a dosage form for systemic delivery, and
wherein the gamma immunoglobulin is administered in combination with
i. an IL-12 nucleic acid expression vector encoding an IL-12 polypeptide,
ii. an IL-12 polypeptide
wherein the IL-12 nucleic acid expression vector or the IL-12 polypeptide is provided by administration into a tumour, into the vicinity of a tumour, or to the lymph node associated with a tumour.

12. The gamma immunoglobulin for use according to claim 11, wherein the gamma immunoglobulin is selected from the group comprising ipilimumab, MDX-1105/BMS-936559, opdivo (MDX-1106/BMS-936558/ONO-4538), MK-3475/SCH 900475 and AMP-224.

13. The gamma immunoglobulin for use according to claim 11 or 12, wherein said IL-12 polypeptide comprises
a. a polypeptide sequence at least 95% identical to the sequence of human p35 (SEQ ID 05), and
b. a polypeptide sequence at least 95% identical to the sequence of human p40 (SEQ ID 06).

14. The gamma immunoglobulin for use according to any one of claim 11 to 13, wherein said malignant neoplastic disease is glioma, glioblastoma multiforme, meningioma, secondary brain cancer, brain metastases, melanoma, pancreatic cancer, lung cancer, prostate cancer or bladder cancer.
